# EUROPEAN PATENT APPLICATION

(11) **EP 4 800 706 A1**
(43) Date of publication of application: **02.09.2026**
(21) Application number: 24882136.5
(22) Date of filing: 07.10.2024
(51) Int. Cl.: G16H 40/60, A61B 5/16, G16H 20/00

(54) **MIND-BODY STATE INTERVENTION APPARATUS, MIND-BODY STATE INTERVENTION SYSTEM, AND MIND-BODY STATE INTERVENTION METHOD**

(30) Priority: 25.10.2023 JP 2023183519
(71) Applicant: Panasonic Intellectual Property Management Co., Ltd., Kadoma-shi, Osaka 571-0057 (JP)
(72) Inventor: KAKIMOTO, Yuki, Kadoma-shi, Osaka 571-0057 (JP); SHIKII, Shinichi, Kadoma-shi, Osaka 571-0057 (JP); YOSHIMURA, Keita, Kadoma-shi, Osaka 571-0057 (JP)
(74) Representative: Appelt, Christian W.
(86) International application number: PCT/JP2024/035736
(87) International publication number: WO 2025/089019

(57) **Abstract**

A mind-body state intervention apparatus (100) includes a physiological amount acquirer (110) that acquires a physiological amount of a subject detected by a physiological amount detector (160); a quantification processor (120) that quantifies, for a predetermined mind-body state, two mind-body states based on the physiological amount, the two mind-body states including a mind-body state that the subject is aware of and a mind-body state that the subject is unaware of; a first storage (130) that stores relational data in advance, the relational data indicating relationships between each of multiple intervention control schemes and intervention effects on the two mind-body states quantified; and an intervention controller (140) that performs intervention control in which, with reference to the relational data, one intervention control scheme is selected from among the multiple intervention control schemes, based on the intervention effect on at least one of the two mind-body states quantified by the quantification processor (120), and intervention equipment (150) is controlled in accordance with the selected intervention control scheme.

## Description

### [Technical Field]

The present disclosure relates to a mind-body state intervention apparatus, a mind-body state intervention system, and a mind-body state intervention method.

### [Background Art]

Examples of human mind-body states including physical and emotional conditions include various feelings such as fatigue, sleepiness, awakening, joy, and anger. Among these feelings, particularly negative mind-body states such as fatigue, sleepiness, and anger are considered necessary to be quantified and subjected to intervention control based on the quantified mind-body states, in order to bring the mind-body states closer to better conditions.

For fatigue, for example, it is considered to measure fatigue levels of office workers in order to prevent losses due to reduced productivity caused by fatigue or to prevent illness-induced fatigue such as depression. In general, quantification technology for fatigue is realized using technology for measuring the autonomic nervous system through analysis of heart rate variability. Examples of the intervention control corresponding to quantified fatigue include supplement intake containing ingredients such as vitamin B1, GABA, and imidazole dipeptide that are said to have fatigue recovery effects, forest bathing that is believed to regulate the autonomic nervous system and have fatigue recovery effects, and listening to music that activates the parasympathetic nervous system.

Meanwhile, for sleepiness, it is considered to measure the sleepiness level of a driver during driving in order to reduce accidents caused by sleepiness. In general, quantification technology for sleepiness is realized using technology for measuring the autonomic nervous system through analysis of heart rate variability. The intervention control according to quantified sleepiness is realized by, for example, warnings via alarm sound, vibration stimulation, and stimulation using awakening scents.

For anger, it is considered to measure the anger levels of office workers in order to prevent deterioration of interpersonal relationships due to anger and thereby improve the work environment. In general, quantification technology for anger is realized using technology for measuring the autonomic nervous system through analysis of heart rate variability. The intervention control according to quantified anger is realized by, for example, playing soothing music, encouraging deep breathing or other kinds of breathing, and stimulation using scents having sedative effects.

Meanwhile, it is known that there is a difference in level between conscious and unconscious mind-body states. In the case of fatigue, for example, there is a difference between fatigue that a subject is conscious of (hereinafter referred to as "subjective fatigue") and fatigue that the subject is unconscious of (hereinafter referred to as "objective fatigue"). The subjective fatigue and the objective fatigue do not necessarily correspond. In some cases, the objective fatigue may be masked by psychological effects such as working motivation or consumption of nutritional drinks and be perceived as the subjective fatigue while the psychological effects on the objective fatigue increase or decrease. In view of this, Patent Literature (PTL) 1 discloses a technique for quantifying the subjective fatigue by posing fatigue-related questions and quantifying the objective fatigue by measuring tremors that are micro-vibrations at specific body locations. PTL 2 discloses a technique for determining the presence or absence of masking of the objective fatigue through analysis of biological signals that are highly relevant to autonomic nervous functions.

For sleepiness, similarly to the example of fatigue, it is known that there is a difference in level between sleepiness that a subject is conscious of (hereinafter, referred to as "subjective sleepiness") and sleepiness that the subject is unconscious of (hereinafter, referred to as "objective sleepiness"). For example, it is thought that light objective sleepiness is likely to be underestimated as the subjective sleepiness. The subjective sleepiness and the objective sleepiness do not necessarily correspond. In some cases, the objective sleepiness may be masked by psychological effects such as consumption of nutritional drinks or tension caused by driving a car or the like and be perceived as the subjective sleepiness.

For anger, similarly to the example of fatigue, it is known that there is a difference in level between anger that a subject is conscious of (hereinafter, referred to as "subjective anger") and anger that the subject is unconscious of (hereinafter, referred to as "objective anger"). For example, when intensely angry, people may lose control and be almost unaware of their feeling as subjective anger. The subjective anger and the objective anger do not necessarily correspond. In some cases, the objective anger may be masked by psychological effects such as individual personality or social standing and be perceived as the subjective anger.

### [Citation List]

### [Patent Literature]

[PTL 1]
   Japanese Unexamined Patent Application Publication No. 2002-191579
[PTL 2]
   Japanese Unexamined Patent Application Publication No. 2018-93997
[PTL 3]
   Japanese Unexamined Patent Application Publication No. 2008-305107

### [Summary of Invention]

### [Technical Problem]

For example, the intervention control schemes described in PTL 3 starts from the one that has a low effect on fatigue. If the intervention effect is insufficient, intervention control is performed so as to gradually increase the intervention effect. This suppresses a decrease in work efficiency while relieving fatigue. If there are four stages of the intervention control schemes, the fourth-stage intervention control scheme is the most effective to achieve the intervention effect when the user's fatigue level is high. However, intervention is performed stepwise starting from the first stage. Thus, the user has to wait until the fourth-stage intervention control scheme begins. That is, these schemes are ineffective due to difficulty in initially selecting the optimal intervention control scheme.

Besides, the selection of the intervention control schemes disclosed in PTL 3 does not take into account the difference between the subjective fatigue and the objective fatigue as described above. Therefore, optimal intervention may not be achieved due to no consideration given to the characteristics of the intervention control schemes relative to the fatigue type, i.e., one intervention control scheme may have a high intervention effect on the subjective fatigue but have a low intervention effect on the objective fatigue.

The invention according to the present application primarily addresses the above issues and aims to provide a mind-body state intervention apparatus, a mind-body state intervention system, and a mind-body state intervention method that provide a more efficient and effective mind-body state intervention control scheme.

### [Solution to Problem]

To solve the above-described objective, one embodiment of the mind-body state intervention apparatus according to the present disclosure includes a physiological amount acquirer that acquires a physiological amount of a subject detected by a physiological amount detector, a quantification processor that quantifies, for a predetermined mind-body state, two mind-body states based on the physiological amount, the two mind-body states including a mind-body state that the subject is aware of and a mind-body state that the subject is unaware of, a storage that stores relational data in advance, the relational data indicating relationships between each of a plurality of intervention control schemes and intervention effects on the two mind-body states quantified, and an intervention controller that performs intervention control in which, with reference to the relational data, one intervention control scheme is selected from among the plurality of intervention control schemes, based on the intervention effect on at least one of the two mind-body states quantified by the quantification processor, and intervention equipment is controlled in accordance with the one intervention control scheme selected.

Moreover, to achieve the above-described objective, one embodiment of the mind-body state intervention system according to the present disclosure includes a mind-body state intervention system that a physiological amount acquirer that acquires a physiological amount of a subject detected by a physiological amount detector, a quantification processor that quantifies, for a predetermined mind-body state, two mind-body states based on the physiological amount, the two mind-body states including a mind-body state that the subject is aware of and a mind-body state that the subject is unaware of, a storage that stores relational data in advance, the relational data indicating relationships between each of a plurality of intervention control schemes and intervention effects on the two mind-body states quantified, and an intervention controller that performs intervention control in which, with reference to the relational data, one intervention control scheme is selected from among the plurality of intervention control schemes, based on the intervention effect on at least one of the two mind-body states quantified by the quantification processor, and intervention equipment is controlled in accordance with the one intervention control scheme selected, the physiological amount detector, and the intervention equipment.

Furthermore, to achieve the above-described objective, one embodiment of the mind-body state intervention method according to the present disclosure is a mind-body state intervention method that is an information processing method to be executed by a computer. The mind-body state intervention method includes acquiring a physiological amount of a subject detected by a physiological amount detector, quantifying, for a predetermined mind-body state, two mind-body states based on the physiological amount, the two mind-body states including a mind-body state that the subject is aware of and a mind-body state that the subject is unaware of, and performing intervention control in which, with reference to relational data that indicates each of a plurality of intervention control schemes and intervention effects on the two mind-body states quantified, one intervention control scheme is selected from among the plurality of intervention control schemes, based on the intervention effect on at least one of the two mind-body states quantified, and intervention equipment is controlled in accordance with the one intervention control scheme selected.

### [Advantageous Effects of Invention]

The mind-body state intervention apparatus according to the present disclosure performs more efficient and effective intervention control by, for a predetermined mind-body state of a subject, distinguishing between the conscious one and the unconscious one and selecting an intervention control scheme based on this distinction.

### [Brief Description of Drawings]

[FIG. 1A]

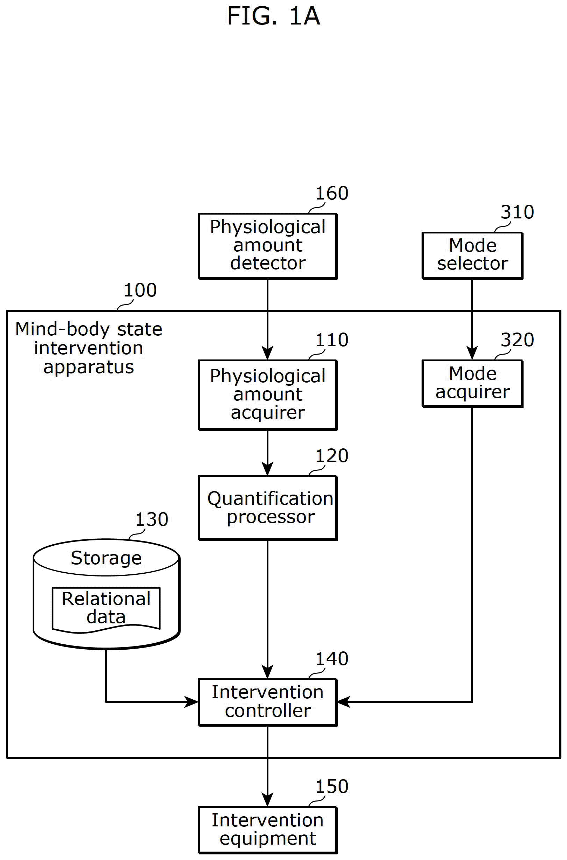

   FIG. 1A is a block diagram showing a characteristic functional configuration of a mind-body state intervention apparatus according to Embodiment 1.
[FIG. 1B]

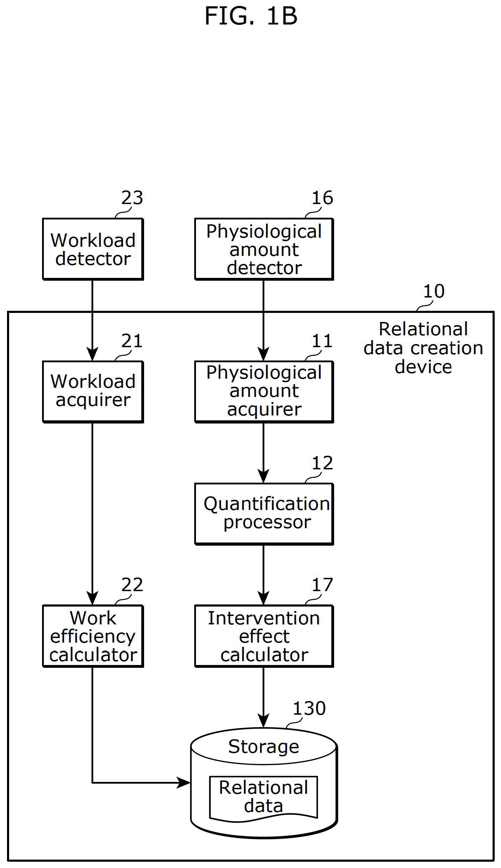

   FIG. 1B is a block diagram showing a configuration of a relational data creation device according to Embodiment 1.
[FIG. 1C]

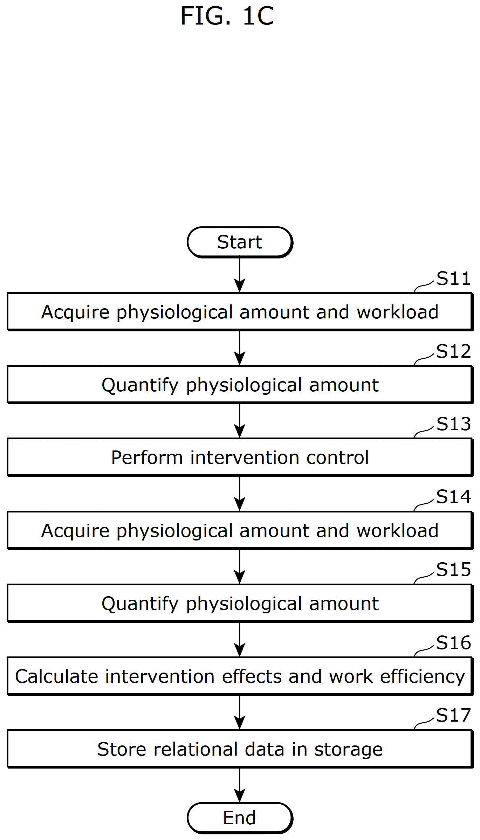

   FIG. 1C is a flowchart showing operations of the relational data creation device according to Embodiment 1.
[FIG. 2A]

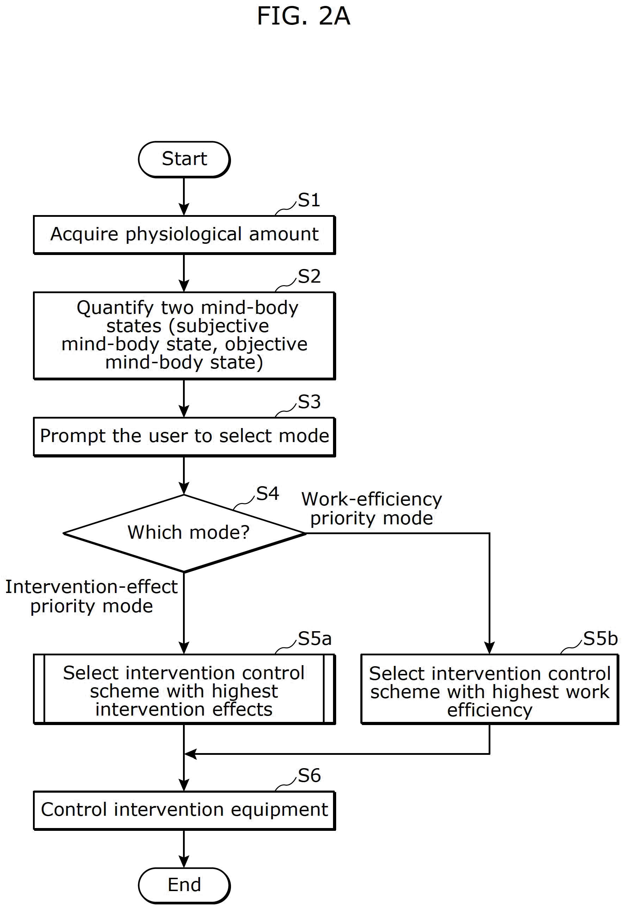

   FIG. 2A is a flowchart showing operations of the mind-body state intervention apparatus according to Embodiment 1.
[FIG. 2B]

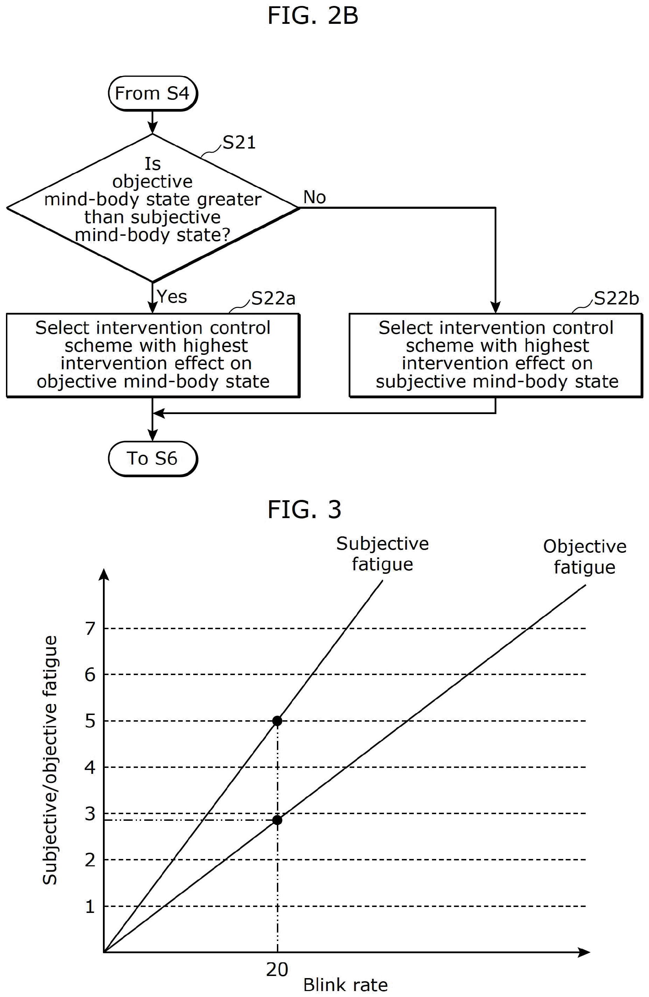

   FIG. 2B is a flowchart showing a detailed operation performed in step S5a in FIG. 2A.
[FIG. 3]
   FIG. 3 shows one example of fatigue functions.
[FIG. 4]

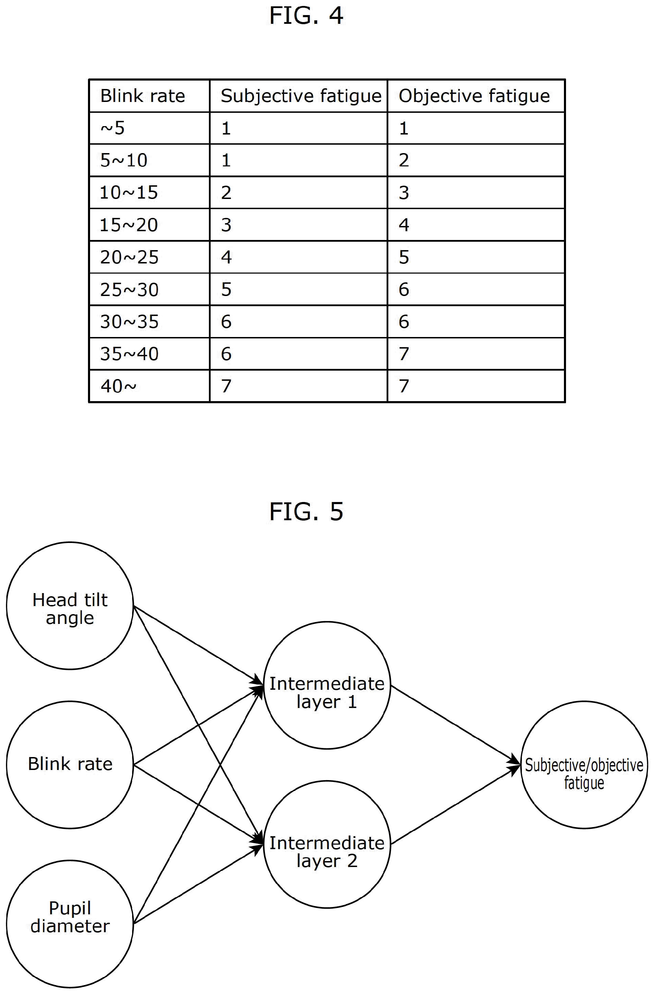

   FIG. 4 shows one example of a physiological amount-fatigue correspondence table.
[FIG. 5]
   FIG. 5 shows one example of a neural network.
[FIG. 6]

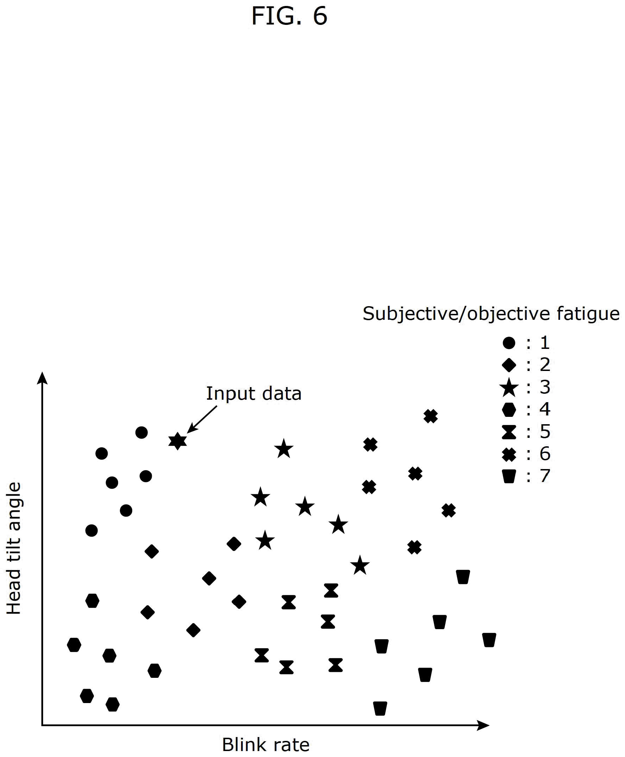

   FIG. 6 shows one example of k-nearest neighbors.
[FIG. 7]

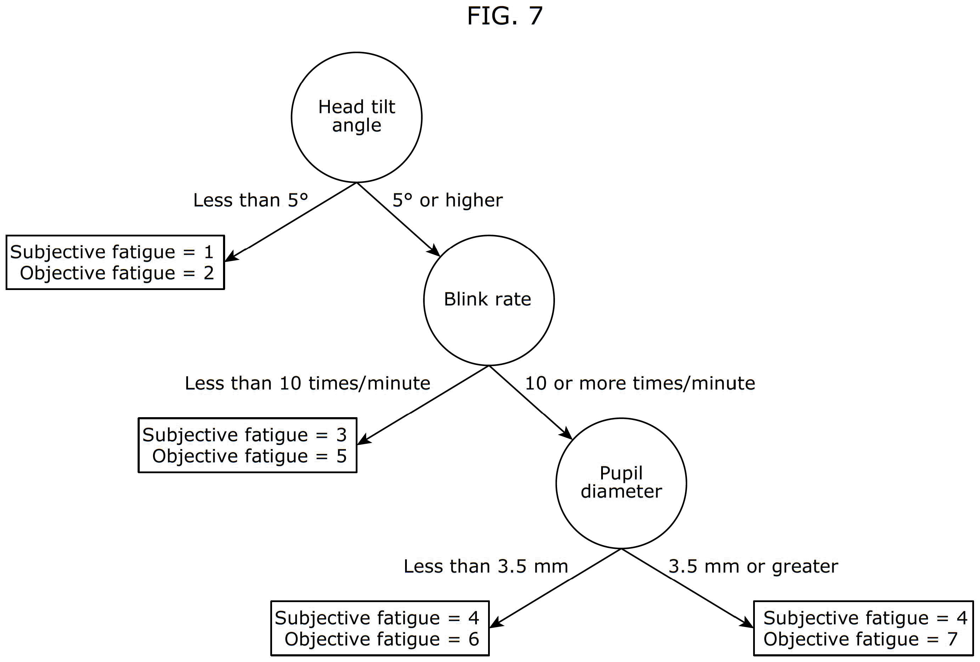

   FIG. 7 shows one example of a random forest model.
[FIG. 8]

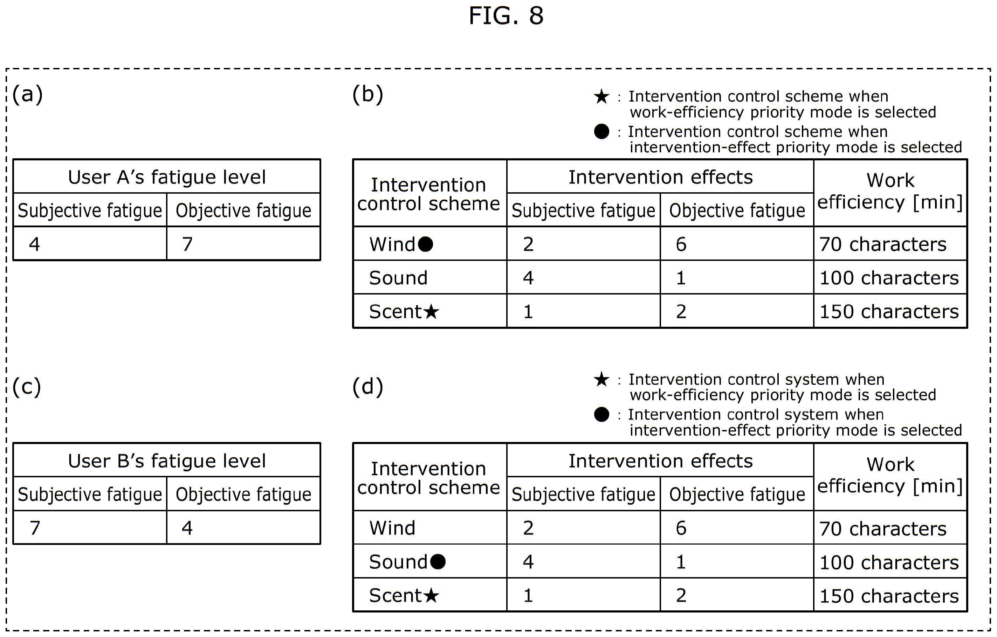

   FIG. 8 shows one example of selecting an intervention control scheme.
[FIG. 9]

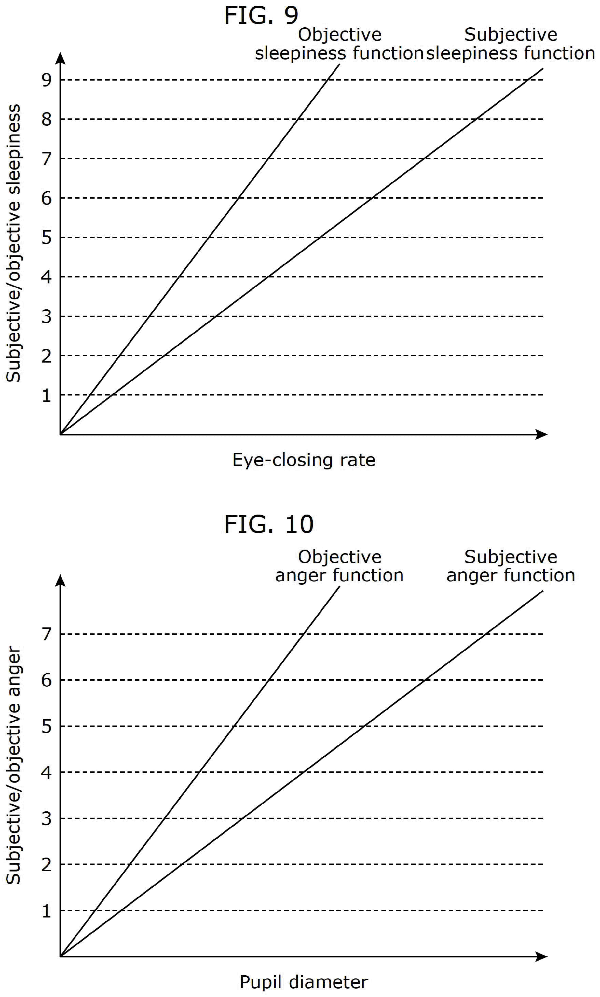

   FIG. 9 shows one example of sleepiness functions.
[FIG. 10]
   FIG. 10 shows one example of anger functions.
[FIG. 11]

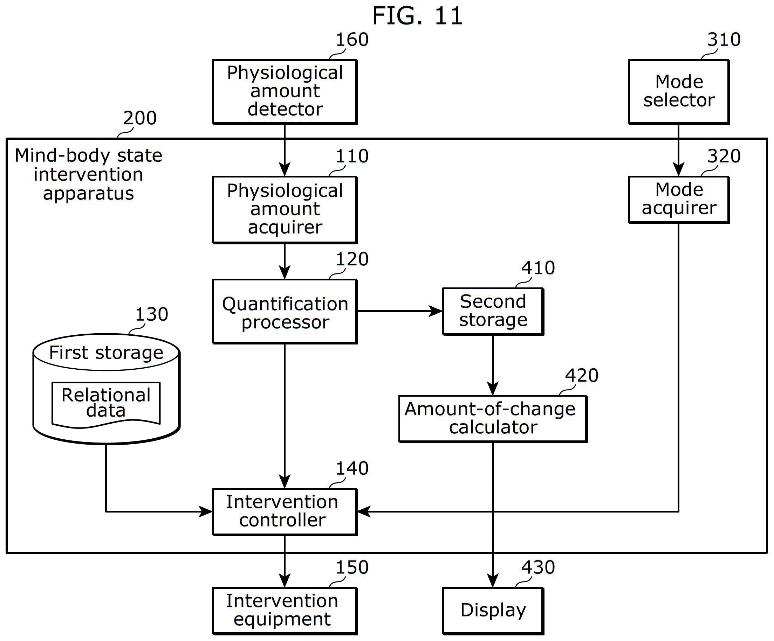

   FIG. 11 is a block diagram showing a functional configuration of a mind-body state intervention apparatus according to Variation 1 of Embodiment 1.
[FIG. 12]

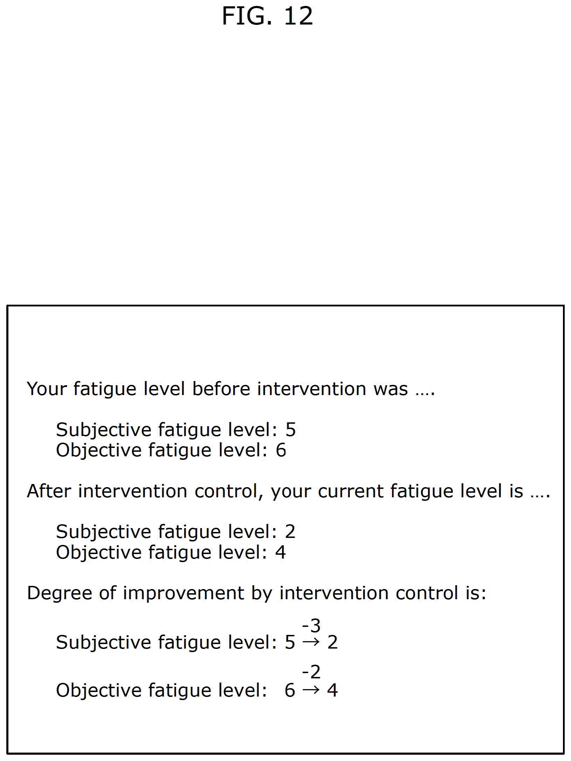

   FIG. 12 shows one example of a display screen according to Variation 1 of Embodiment 1.
[FIG. 13]

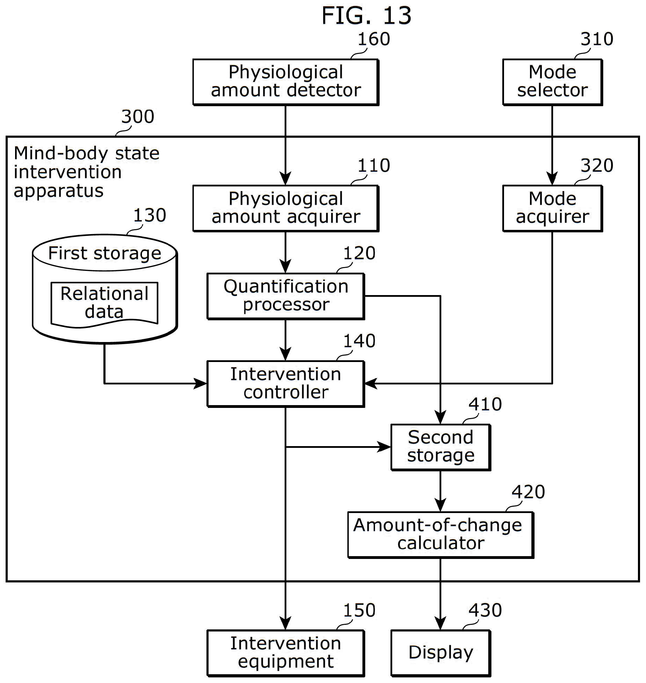

   FIG. 13 is a block diagram showing a functional configuration of a mind-body state intervention apparatus according to Variation 2 of Embodiment 1.
[FIG. 14]

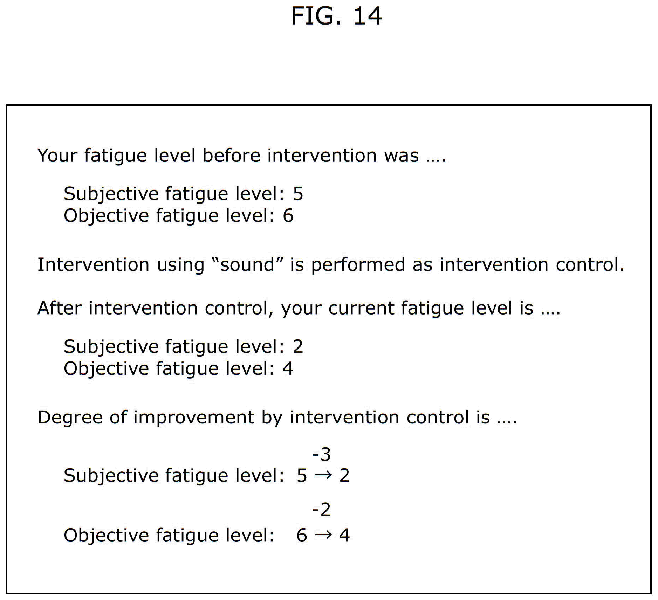

   FIG. 14 shows one example of a display screen according to Variation 2 of Embodiment 1.
[FIG. 15]

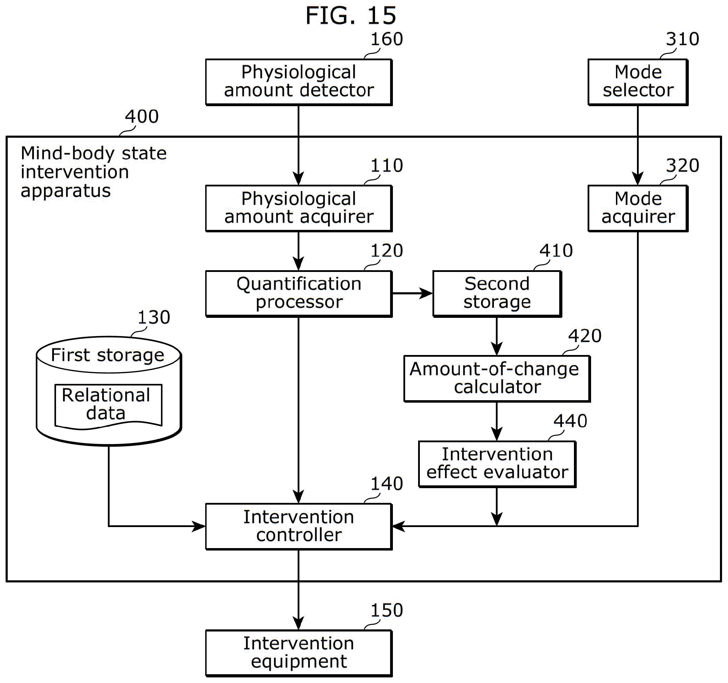

   FIG. 15 is a block diagram showing a characteristic functional configuration of a mind-body state intervention apparatus according to Embodiment 2.
[FIG. 16]

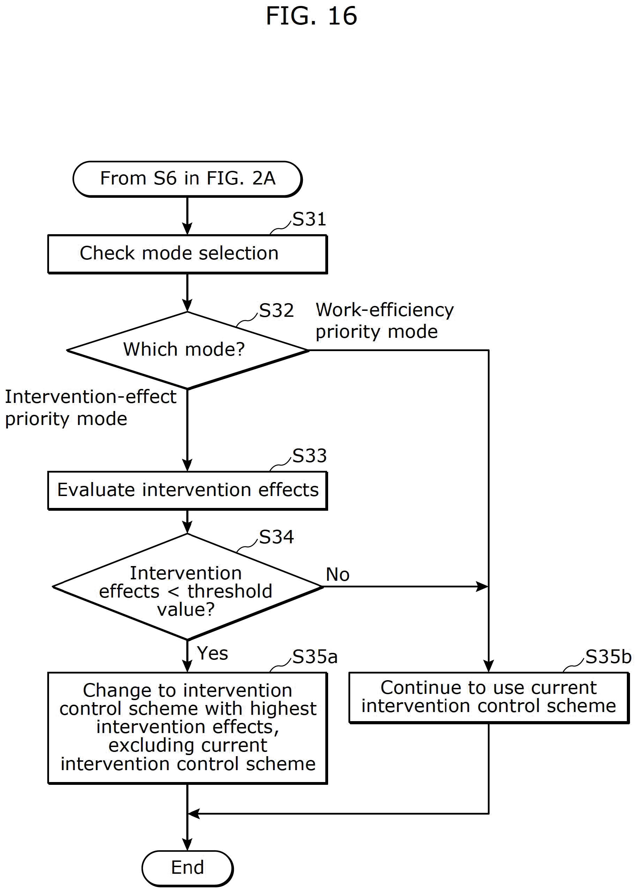

   FIG. 16 is a flowchart for describing operations of the mind-body state intervention apparatus according to Embodiment 2.
[FIG. 17]

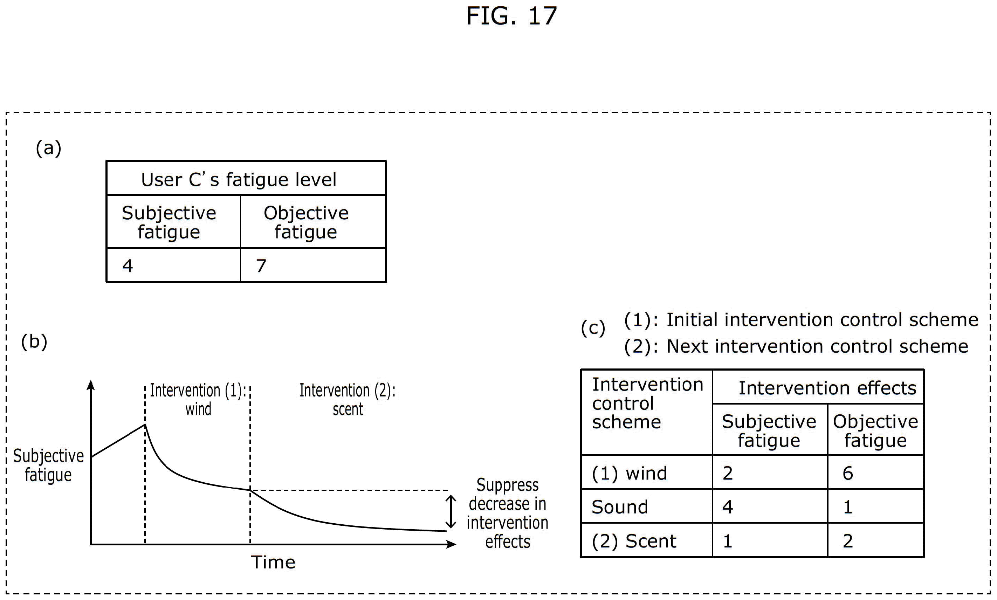

   FIG. 17 shows one example of changing the intervention control scheme according to Embodiment 2.
[FIG. 18]

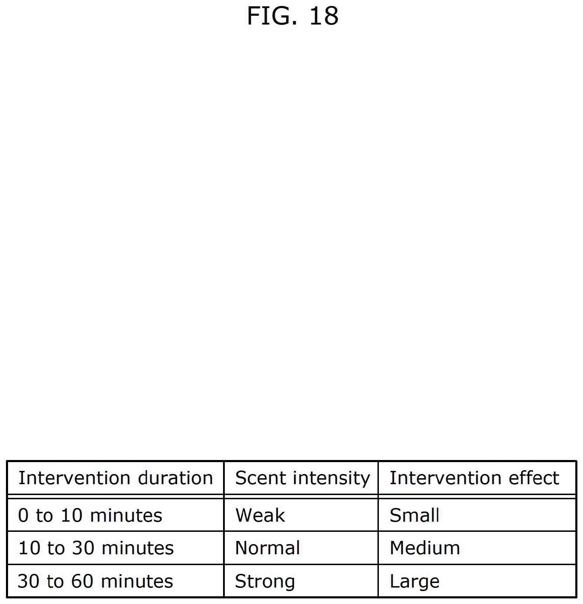

   FIG. 18 shows one example of stepwise intervention control according to Variation 1 of Embodiment 2.
[FIG. 19]

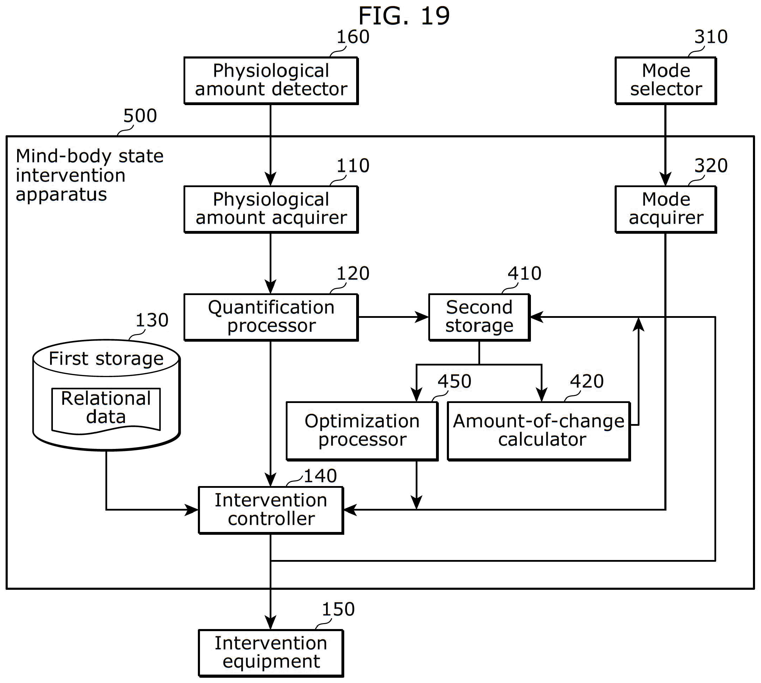

   FIG. 19 is a block diagram showing a characteristic functional configuration of a mind-body state intervention apparatus according to Embodiment 3.
[FIG. 20]

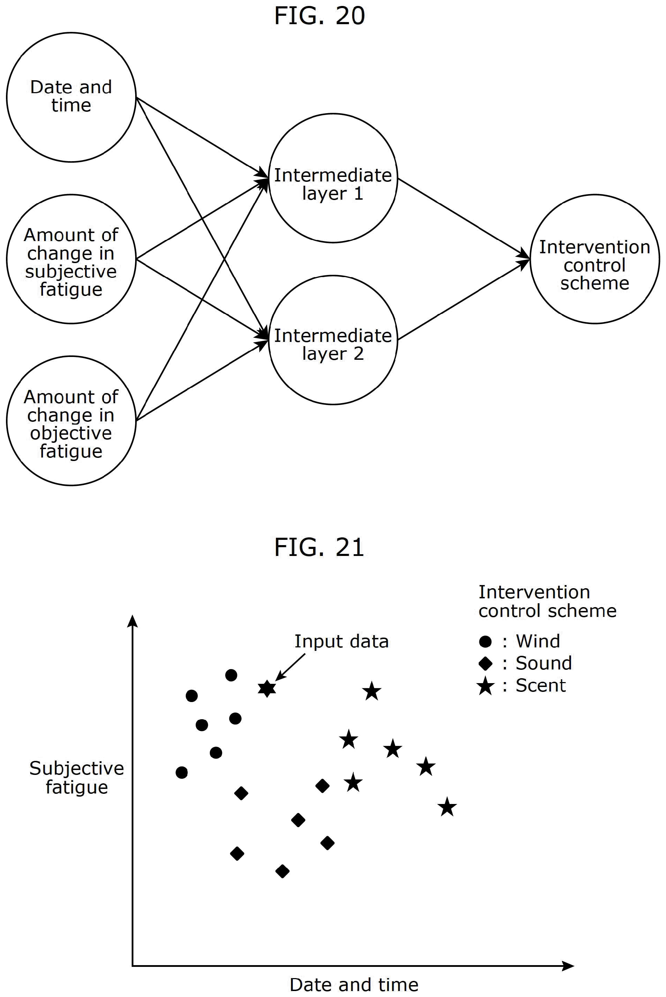

   FIG. 20 shows one example of a neural network in an optimization technique according to Embodiment 3.
[FIG. 21]
   FIG. 21 shows one example of k-nearest neighbors in the optimization technique according to Embodiment 3.
[FIG. 22]

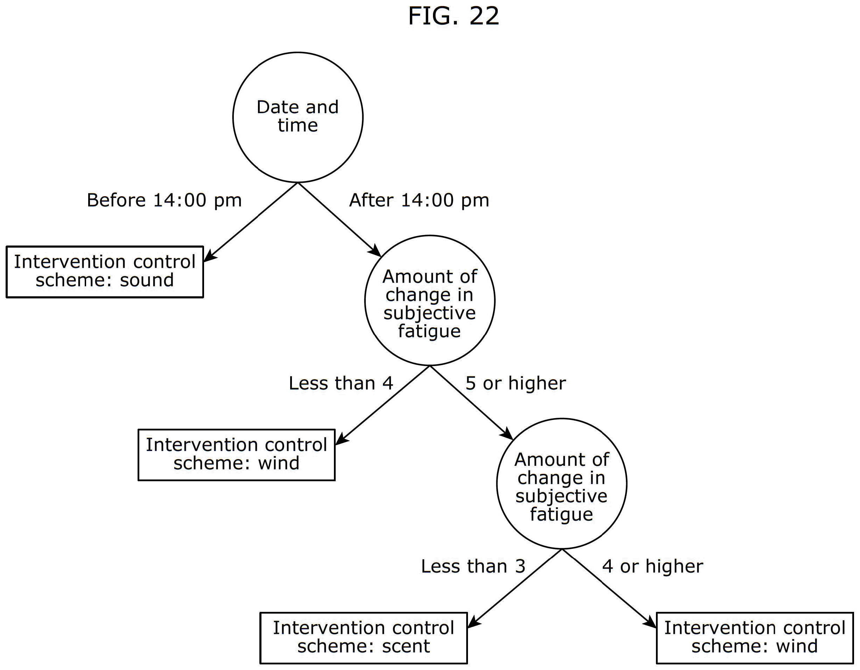

   FIG. 22 shows one example of random forest in the optimization technique according to Embodiment 3.
[FIG. 23]

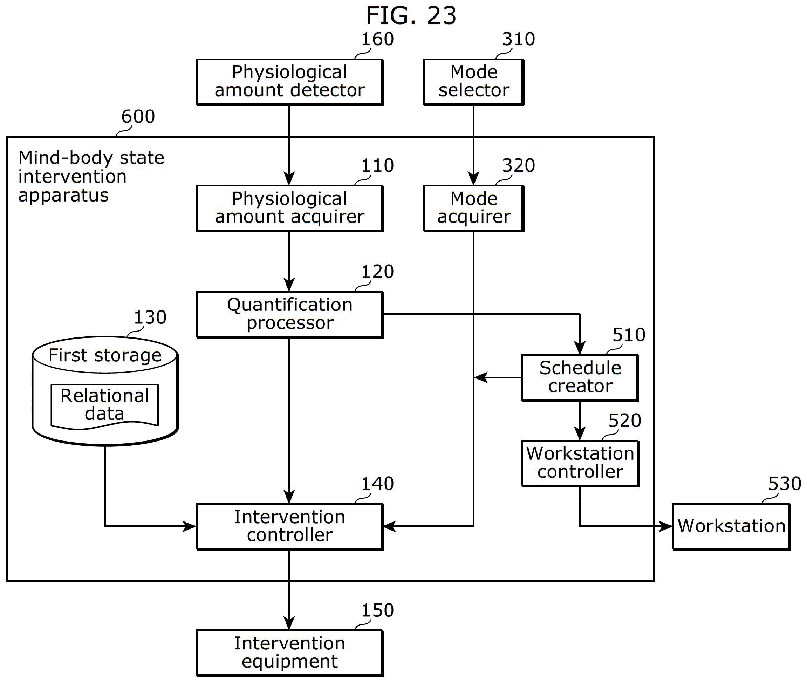

   FIG. 23 is a block diagram showing a characteristic functional configuration of a mind-body state intervention apparatus according to Embodiment 4.
[FIG. 24]

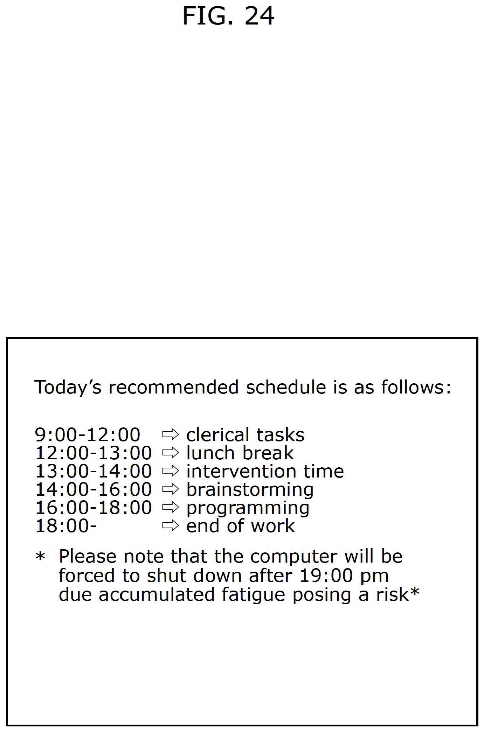

   FIG. 24 shows one example of a schedule.
[FIG. 25]

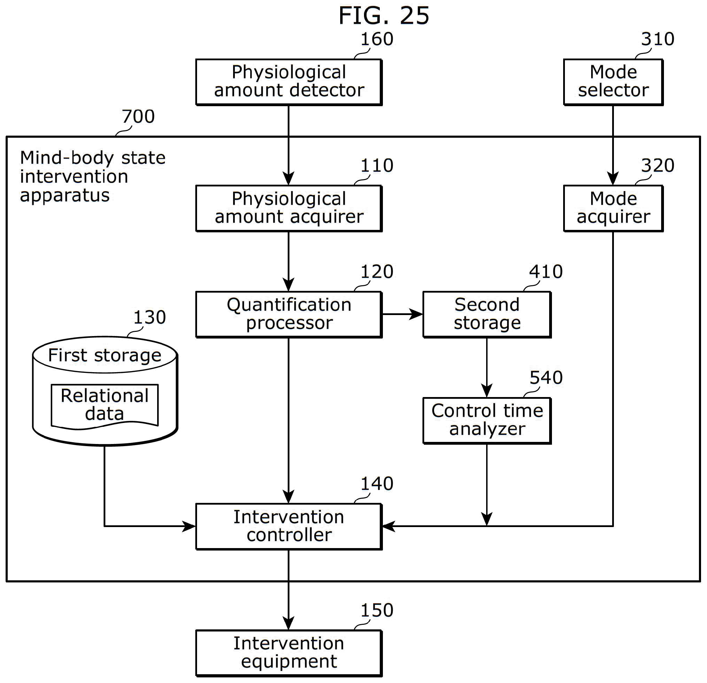

   FIG. 25 is a block diagram showing a characteristic functional configuration of a mind-body state intervention apparatus according to Embodiment 5.
[FIG. 26]

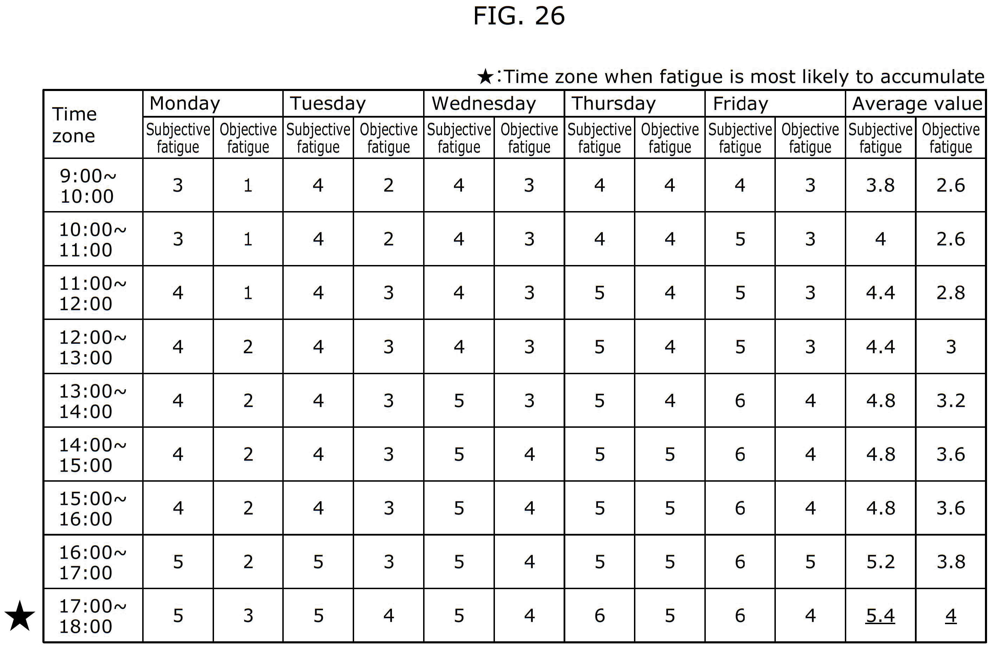

   FIG. 26 shows one example of analyzing time zones where fatigue is likely to accumulate.
[FIG. 27]

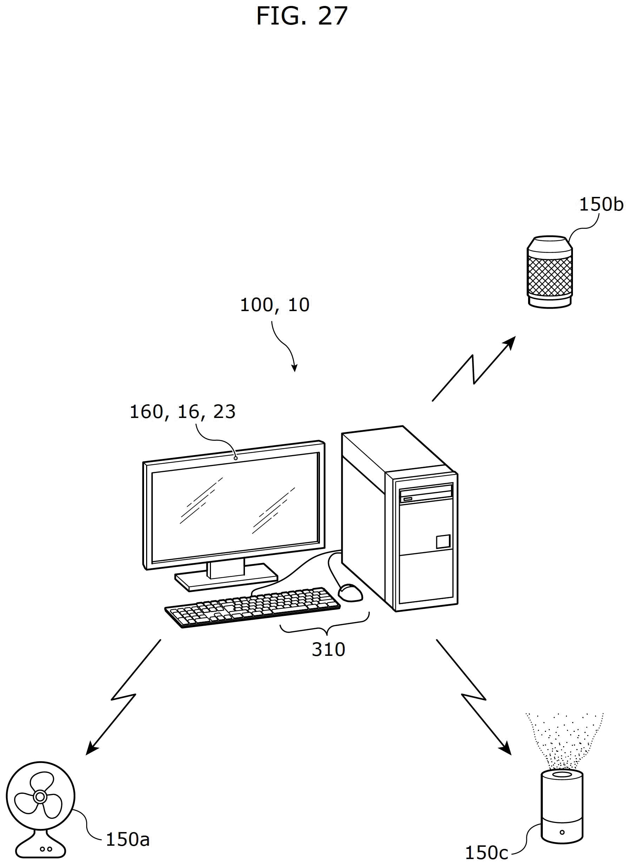

   FIG. 27 is an outside drawing of a mind-body state intervention system.

### [Description of Embodiments]

Embodiments of a mind-body state intervention apparatus according to the present invention are described hereinafter with reference to the drawings. Note that identical elements are given the same reference signs, and their description may be omitted. To facilitate understanding of the drawings, each constituent element is schematically shown primarily. Each embodiment described below shows one specific example of the present invention. Numerical values, shapes, constituent elements, steps, a sequence of steps, and so on illustrated in the following embodiments are mere examples, and do not intend to limit the scope of the present invention. Among the constituent elements in the following embodiments, those that are not recited in any independent claim, which indicates the broadest concept, are described as arbitrary constituent elements. Each of the contents described in all of the embodiments may be used in combination. Similarly, configurations described in variations of the embodiments may also be used in combination.

### [Embodiments]

### [Embodiment 1]

A mind-body state intervention apparatus according to Embodiment 1 of the present invention is described with reference to FIGS. 1A to 10. The description here firstly uses fatigue as one example of a mind-body state.

### [Configuration]

FIG. 1A is a block diagram showing a characteristic functional configuration of the mind-body state intervention apparatus according to Embodiment 1. Mind-body state intervention apparatus 100 is an apparatus that provides a more efficient and effective intervention control scheme by distinguishing between a subjective mind-body state and an objective mind-body state and then selecting an intervention control scheme based on this distinction. Mind-body state intervention apparatus 100 includes physiological amount acquirer 110, quantification processor 120, first storage 130, intervention controller 140, and mode acquirer 320 and communicates with intervention equipment 150, physiological amount detector 160, and mode selector 310. Mind-body state intervention apparatus 100 may further include constituent elements other than those described above. Intervention as used herein refers to processing that affects the mind-body state of a subject. The subjective mind-body state refers to a mind-body state that the subject is conscious of, and the objective mind-body state refers to a mind-body state that the subject is unconscious of.

Physiological amount detector 160 is a device that acquires a physiological amount. The physiological amount refers to information about each individual's unique physical characteristics, such as face, electrocardiogram, and voice print. Physiological amount detector 160 is, for example, a camera mounted on a personal computer. The camera captures an image of a subject (hereinafter, simply referred to as a "person") who is operating the personal computer. Then, the physiological amount is acquired from the person's face image captured by the camera. For example, in the case where physiological amount detector 160 is a camera, the physiological amount includes, for example, blink rate, pupil diameter, and skin color that are acquired within a fixed period of time.

The blink rate can be measured as follows. First, the face image captured by the camera is binarized through threshold processing, and edges of the face image are extracted to detect eye contours and to set eye regions. For each frame, the eye regions in the face image are scanned to determine the areas of the eye regions. When a blink occurs, these areas become zero. Thus, the blink count can be acquired by counting the number of times the areas become zero. The pupil diameter is acquired by binarizing the face image captured by the camera through threshold processing and extracting edges of the face image to detect pupil contours and to measure widths between edges. The skin color is acquired from RGB values at predetermined positions or in predetermined regions in the face image captured by the camera.

Other than a camera, physiological amount detector 160 may also be, example, a heart rate sensor or a microphone. Physiological amount detector 160 is not limited to a camera.

In the case of using a heart rate sensor, the physiological amount refers to, for example, LF or HF of the heart rate variability, the heart rate, or the respiration rate acquired by the heart rate sensor. First, time-series data on heart-rate interval variability (hereinafter, referred to as "RRI data") is acquired by the heart rate sensor. LF and HF are values obtained by calculating power spectrums of the acquired RRI data and integrating the power spectrums of the LF (from 0.05 Hz to 0.15 Hz) and the power spectrum of the HF (from 0.15 Hz to 0.40 Hz). The heart rate refers to the number of peaks detected in the RRI data per minute. The respiration rate can be estimated using the heart rate variability caused by respiratory sinus arrhythmia (hereinafter, referred to as "RSA"). Specifically, RSA generally refers to a phenomenon in which breathing-in increases the heart rate and breathing-out reduces the heart rate. That is, if the interval between peaks in the RRI data is defined as the heart rate interval, breathing-in shortens the heart rate interval, and breathing-out causes variations in the heart rate, such as lengthening the heart rate interval. Thus, the respiration rate can be estimated from the number of peaks detected in the heart rate variability.

In the case of using a microphone, the physiological amount refers to, for example, the volume, pitch, and formant frequency of acquired voice. First, voice data acquired by the microphone is subjected to Fourier transform to acquire a frequency spectrum. The volume and pitch of the voice are represented by the amplitude and frequency of the acquired frequency spectrum. The formant frequency corresponds to the lowest frequency that is calculated from an autocorrelation function of the voice data acquired by the microphone and at which a peak of the autocorrelation function is detected. The formant frequency represents the timbre of the voice. The physiological amount may of course be something other than the above, and the configuration of physiological amount detector 160 is not specified here.

Physiological amount acquirer 110 communicates with physiological amount detector 160 and acquires the physiological amount detected by physiological amount detector 160. For example, physiological amount acquirer 110 may be a communication interface, a program for acquiring the physiological amount, or a processor for executing the program.

Quantification processor 120 quantifies the subjective fatigue and the objective fatigue, based on the physiological amount acquired by physiological amount acquirer 110.

Here, a technique for quantifying the subjective fatigue and the objective fatigue is described. One example of the quantification technique is using fatigue functions. The fatigue functions refer to functions that take the physiological amount as input and output subjective fatigue and objective fatigue that have been quantified. By way of example, fatigue functions using the blink rate shown in FIG. 3 are described here. Functions for each of the subjective fatigue and the objective fatigue with respect to the blink rate are created in advance through experiment. Examples of method of acquiring the subjective fatigue include the visual analog scale (hereinafter, referred to as "VAS"), the numerical rating scale (hereinafter, referred to as "NRS"), and the face scale (or face rating scale). In these methods, a position on the scale or a value indicated in a chart by a person is acquired as a quantified value. The method of acquiring the objective fatigue is acquiring the physiological amount such as the amylase content, the cortisol content, or the human herpesviruses content in saliva. In this method, a value obtained by replacing the physiological amount with an indicator used in the acquisition of the subjective fatigue is acquired as a quantified value.

Then, the fatigue functions are created using the quantified values of the subjective fatigue and the objective fatigue acquired in advance through experiment and the physiological amount acquired by physiological amount detector 160. As one example, FIG. 3 shows the fatigue functions when the physiological amount is the blink rate and the degrees of the subjective fatigue and the objective fatigue are quantified as values expressed on a seven-level equidistant scale. The equidistant scale allows the subjective fatigue and the objective fatigue to be quantified using a predetermined number of level values. Here, the higher the level value, the greater the degree of fatigue. For example, Level 1 indicates no fatigue at all, and Level 7 indicates extreme physical and mental exhaustion to a point of not wanting to work any more. Although the degree of fatigue has been described using the above example, the way of expressing the fatigue level is not limited thereto. The subjective fatigue and the objective fatigue can be quantified by taking the blink rate acquired by the physiological amount detector as an input value of the fatigue functions. For example, in the case where the blink rate acquired by the physiological amount detector is 20 times , the objective fatigue is at Level 5 as shown in FIG. 3. The subjective fatigue is at 2.8, which is not an integer value, but is rounded off to Level 3. Note that the above example describes a case where when the output value that is not an integer value is converted by rounding, but the conversion method is not limited thereto; the output value may be rounded down to the nearest integer.

The above has described, as one example, a method using the fatigue functions when the physiological amount is the blink rate and the degrees of the subjective fatigue and the objective fatigue are quantified as values expressed on a seven-level equidistant scale. However, this does not intend to limit the method of acquiring the subjective fatigue and the objective fatigue and the physiological amount used as a input value.

Other than using the fatigue functions the technique for quantifying the subjective fatigue and the objective fatigue may be using a physiological amount-fatigue correspondence table or machine learning. The technique for quantifying the subjective fatigue and the objective fatigue is not limited to using the fatigue functions.

In the case of using a physiological amount-fatigue correspondence table, the subjective fatigue and the objective fatigue are quantified based on the magnitude of the physiological amount acquired by the physiological amount detector, based on the correspondence table that associates the physiological amount in advance with the subjective fatigue and the objective fatigue. As one example, FIG. 4 shows the physiological amount-fatigue correspondence table where the physiological amount is the blink rate and the degree of fatigue is expressed on a seven-level equidistant scale. The methods of acquiring the subjective fatigue and the objective fatigue are the same as those described as one example in the case of the fatigue functions, and therefore redundant description is omitted here.

In the case of using machine learning, one example is using a neural network . In the case of using a neural network, as shown in FIG. 5, at least one physiological amount acquired by the physiological amount detector is used as a feature, and the subjective fatigue and the objective fatigue are quantified using a created machine learning model. Other then using a neural network, the machine learning may also use k-nearest neighbors or random forest. In the k-nearest neighbors or the random forest, as shown in FIGS. 6 and 7, at least one physiological amount acquired by the physiological amount detector is used as a feature as in the case of using a neural network, and the subjective fatigue and the objective fatigue are quantified using a created machine learning model. Here, the machine learning technique is not specified. The physiological amount used in the quantification of the subjective fatigue and the objective fatigue may also be any other physical amount.

In the present embodiment, physiological amount detector 160 calculates the physiological amount by using data that indirectly indicates the physiological amount (e.g.,. a moving image showing eyes of the subject), and physiological amount acquirer 110 and quantification processor 120 acquire the calculated physiological amount. Alternatively, the physiological amount acquired by physiological amount acquirer 110 and quantification processor 120 may be the data that indirectly indicates the physiological amount, and the calculation of the physiological amount using data that indirectly indicates the physiological amount may be conducted by physiological amount acquirer 110 or quantification processor 120.

First storage 130 stores relational data created in advance. The relational data refers to data that indicates the relationships between each of multiple intervention control schemes and intervention effects on the subjective fatigue and the objective fatigue that have been quantified, and the relationships between each of the multiple intervention control schemes and work efficiency. The intervention effects refer to the amounts of change in the subjective fatigue and the objective fatigue felt by the subject before and after intervention. The work efficiency refers to the amount of change in workload per unit time before and after intervention control within an intervention time. Methods of calculating the intervention effects and the work efficiency and a procedure for creating the relational data will be described later. First storage 130 is, for example, read-only memory (ROM), random access memory (RAM), a hard disk drive (HDD), or a solid state drive (SSD). Here, the storage is not specified.

Mode selector 310 prompts a user to select a mode that serves as an indicator in selecting the intervention control scheme. Examples of the mode include "Intervention Effect Mode" that prioritizes the intervention effects and "Work Efficiency Mode" that prioritizes the work efficiency. Mode selector 310 is realized by, for example, a touch panel, a mouse, and a keyboard.

Mode acquirer 320 acquires the mode selected by the user from mode selector 310.

Intervention controller 140 references the relational data stored in first storage 130 to select an appropriate intervention control scheme from among the multiple intervention control schemes, based on the intervention effects on the subjective fatigue and the objective fatigue that have been quantified, the work efficiency, and the mode acquired by mode acquirer 320.

Note that quantification processor 120, intervention controller 140, and mode acquirer 320 are realized by a program and a processor that executes the program.

Intervention equipment 150 performs intervention based on the intervention control scheme selected by intervention controller 140. Intervention equipment 150 is, for example, an air conditioner or an electric fan. The air conditioner that has received a signal from intervention controller 140 exposes a person to a wind that is effective against fatigue. The wind effective against fatigue is generally a wind that causes the parasympathetic nervous system to become dominant by exposure and that is characterized by having sedative effects. Examples of the wind effective against fatigue include a wind with 1/f fluctuations, a wind that simulates a natural breeze, and a wind with fluctuations in temperature. Here, the wind effective against fatigue is not specified. Other than an air conditioner, intervention equipment 150 may also be, for example, audio equipment or a scent generator. Here, intervention equipment 150 is given as one example, but it is not limited to an air conditioner.

In the case of using audio equipment, a sound that is effective against fatigue is produced. The sound effective against fatigue is generally a sound that causes the parasympathetic nervous system to become dominant and that is characterized by having sedative effects. Examples of the sound include bird chirping sounds, forest ambient sounds, and hypersonic sounds.

Here, hypersonic sounds refer to sounds that contain high-frequency sounds beyond the human audible range. It has been confirmed that simultaneous reception of hypersonic sounds and audible-range sounds at body surfaces increases alpha waves that are observed when the parasympathetic nervous system is dominant. By exposing people to a sound that contains hypersonic sounds via a loudspeaker, intervention control for fatigue can be performed. Here, bird chirping sounds or the like are provided as one example of the sound effective against fatigue, but the sound effective against fatigue is not limited thereto.

In the case of using a scent generator, a scent effective against fatigue is released to people. The scent effective against fatigue is generally a scent that cause the parasympathetic nervous system to become dominant by exposure and that is characterized by having sedative effects. Examples of the scent include a grapefruit scent, a cypress scent, and a lavender scent. Here, the scent effective against fatigue is not specified.

FIG. 1B is a block diagram showing a configuration of relational data creation device 10 according to Embodiment 1. Relational data creation device 10 is a device that creates, in advance, the relational data that is stored in mind-body state intervention apparatus 100 shown in FIG. 1A. The relational data is created from multiple subjects as targets and stored in first storage 130. Relational data creation device 10 includes physiological amount acquirer 11, quantification processor 12, intervention effect calculator 17, workload acquirer 21, work efficiency calculator 22, and first storage 130 and communicates with physiological amount detector 16 and workload detector 23.

Physiological amount acquirer 11, quantification processor 12, and physiological amount detector 16 have the same functional configurations and perform the same operations as physiological amount acquirer 110, quantification processor 120, and physiological amount detector 160 of mind-body state intervention apparatus 100, and therefore their description is omitted here.

Intervention effect calculator 17 calculates, for each of multiple intervention control schemes, intervention effects on the subjective fatigue and the objective fatigue after intervention control, which have been quantified by quantification processor 12, and stores the relationships between each of the intervention control schemes and the intervention effects as the relational data in storage 130.

Workload acquirer 21 communicates with workload detector 23 to acquire information about a workload of the subject detected by workload detector 23. For example, workload acquirer 21 may be a communication interface, or may be a program for acquiring workload and a processor for executing the program.

Workload detector 23 is, for example, a personal computer. In document preparation using a personal computer, the number of characters entered per minute serves as a workload. For tasks other than document preparation, the number of input steps per minute may serve as a workload for programming tasks, or the number of pages turned per minute may serve as a workload for document reading. In any case, the workload can be detected from a computer work log.

Although a personal computer is used as an example, the workload detector may also be a camera or a weight sensor other than a personal computer.

In the case of a camera, for example, the number of characters written on paper within a predetermined period of time is used as a workload for document preparation. The camera may be placed to capture the paper and to detect the workload from the amount of increase in text captured by the camera within a predetermined period of time. For tasks other than document preparation, the number of times samples are measured within a predetermined period of time or the number of times baggage is moved within a predetermined period of time during baggage transport may be used as a workload for evaluation or measurement.

In the case where the number of times samples are measured within a predetermined period of time is used as a workload, for example, if the samples are placed and measured at a specific installation position, the camera may be placed to capture this sample installation position, and the number of times the samples are left in the installation position for a fixed period of time or more within a predetermined time interval may be counted to detect the workload.

In the case where the number of times people move baggage within a predetermined period of time is used as a workload, the camera may be placed to capture the baggage movement route, and the number of times people move back and forth may be counted to detect the workload.

In the case of a weight sensor, for example, the amount of change in the weight of baggage stored within a predetermined of time is used as a workload for goods transportation. The workload can be detected by mounting the weight sensor on a track bed or on a case in which baggage is stored during transportation and then measuring the amount of change in weight within a predetermined period of time. In this case, the workload increases as more stored baggage is moved out and the weight becomes smaller. In tasks other than goods transportation, for example, the amount of change in the weight of manufactured products may be used as a workload at a manufacturing site, or the amount of change in the weight of waste scheduled for sorting may be used as a workload at a waste sorting site.

In the case where the amount of change in the weight of manufactured products is used as a workload, for example, the manufactured products may be stored in a predetermined place within a predetermined period of time, and a total weight of the stored products may be measured by the weight sensor to detect the workload. Similarly, in the case where the amount of change in the weight of waste scheduled for sorting is used as a workload, for example, the weight of the waste scheduled for sorting may be measured by the weight sensor to detect the workload.

As long as it is an indicator that represents a workload, the workload is not limited to the above examples.

The configuration of workload detector 23 is not specified here.

Work efficiency calculator 22 calculates, for each of multiple intervention control schemes, work efficiency based on the workload acquired by workload acquirer 21, and stores the relationships between each of the intervention control schemes and the work efficiency as the relational data in first storage 130.

First storage 130 also serves as first storage 130 provided in mind-body state intervention apparatus 100 shown in FIG. 1A and stores, as the relational data, data that indicates the relationships between each of multiple intervention control schemes and the intervention effects calculated by intervention effect calculator 17 and the relationships between each of the multiple intervention control schemes and the work efficiency calculated by work efficiency calculator 22.

### [Operations]

Next, operations of the relational data creation device with the above-described configuration according to the present embodiment is described with reference to the flowchart shown in FIG. 1C.

FIG. 1C is a flowchart showing the operations of the relational data creation device according to Embodiment 1.

First, physiological amount acquirer 11 and workload acquirer 21 acquire the physiological amount and workload of a subject detected respectively by physiological amount detector 16 and workload detector 23 (S11 in FIG. 1C). Physiological amount acquirer 11 and workload acquirer 21 may be one device and is, for example, a camera of a personal computer. In the case of using a camera of a personal computer, the physiological amount to be acquired is, for example, the number of blinks, and the workload is, for example, the number of characters written by the subject.

Quantification processor 12 quantifies the subjective fatigue and objective fatigue felt by the subject based on the physiological amount acquired by physiological amount acquirer 11 (S12 in FIG. 1C), and then performs intervention control of the subject using a given intervention control scheme (S13 in FIG. 1C).

After the intervention control, physiological amount acquirer 11 and workload acquirer 21 again acquire the physiological amount and workload of the subject detected respectively by physiological amount detector 16 and workload detector 23 (S14 in FIG. 1C), and quantification processor 12 quantifies the subjective fatigue and objective fatigue felt by the subject based on the physiological amount acquired by physiological amount acquirer 11 (S15 in FIG. 1C).

Intervention effect calculator 17 calculates intervention effects based on the subjective fatigue and objective fatigue felt by the subject acquired and quantified in S12 and S15 in FIG. 1C, and work efficiency calculator 22 calculates the work efficiency based on the workload acquired in S11 and S14 in FIG. 1C (S16 in FIG. 1C).

The intervention effect as used herein refers to the amount of change in subjective fatigue before and after intervention control or the amount of change in objective fatigue before and after intervention control. That is, value A = value B - value C is satisfied, where value A indicates the intervention effect, value B indicates the subjective or objective fatigue before intervention control, and value C indicates the subjective or objective fatigue after intervention control. Specifically, the greater the amount of change in the subjective or objective fatigue before and after intervention control, the higher the intervention effect, i.e., the higher the fatigue recovery effect.

Although the intervention effect is expressed as the amount of change in fatigue before and after intervention control, it may be expressed not only as the amount of change but also as the rate of change or the amount of change per unit time Here, the intervention effect is not limited to being expressed as the amount of change in fatigue before and after intervention control.

In the case where the intervention effect is expressed as the rate of change, value A = value C /value B is satisfied, where value A indicates the intervention effect, value B indicates the fatigue before intervention control, and value C indicates the fatigue after intervention control. Specifically, the lower the rate of change in fatigue before and after intervention control, the higher the intervention effect, i.e., the higher the fatigue recovery effect.

In the case where the intervention effect is expressed as the amount of change per unit time, value A = (value B - value C)/value D value is satisfied, where value A indicates the intervention effect, value B indicates the fatigue before intervention control, value C indicates the fatigue after intervention control, and value D indicates the intervention time. Specifically, the higher the rate of change in subjective or objective fatigue per unit time before and after intervention control, the higher the intervention effect, i.e., the higher the fatigue recovery effect. The fatigue as used herein may be either the subjective fatigue or the objective fatigue.

The work efficiency as used herein is expressed as the amount of change in workload per unit time before and after intervention control within the intervention time. That is, value E = (value G - value F)/value H is satisfied, where value E indicates the work efficiency, value F indicates the workload before intervention control, value G indicates the workload after intervention control, and value H indicates the intervention time.

Although the work efficiency is described as the amount of change in workload per unit time before and after intervention control within the intervention time, the work efficiency may be expressed not only as the amount of change in workload per unit time before and after intervention control within the intervention time, but also as the amount of change in workload before and after intervention control, or the rate of change in workload before and after intervention control. The work efficiency is not limited to being expressed as the amount of change in workload before and after intervention control within the intervention time.

In the case where the work efficiency is expressed as the amount of change in workload before and after intervention control, value E = value G - value F is satisfied, where value E indicates the work efficiency, value F indicates the workload before intervention control, and value G indicates the workload after intervention control.

In the case where the work efficiency is expressed as the rate of change in workload before and after intervention control, value E = value G/value F is satisfied, where value E indicates the work efficiency, value F indicates the workload before intervention control, and value G indicates the workload after intervention control.

Finally, intervention effect calculator 17 and work efficiency calculator 22 store, as the relational data in first storage 130, the relationships between the intervention control scheme and the intervention effects on the subjective fatigue and the objective fatigue and the relationships between the intervention control scheme and the work efficiency (S17 in FIG. 1C).

This procedure (S11 to S17 in FIG. 1C) is repeated while changing the intervention control scheme so as to create the relational data.

Next, operations of the mind-body state intervention apparatus according to the present embodiment are described with reference to FIGS. 2A and 2B. The description here uses a camera as one example of the physiological amount detector, and uses a blink rate, a pupil diameter, or skin color as one example of the physiological amount.

FIG. 2A is a flowchart for the mind-body state intervention apparatus according to Embodiment 1. First, physiological amount acquirer 110 communicates with physiological amount detector 160 and acquires the physiological amount detected by physiological amount detector 160 (S1 in FIG. 2A). Then, quantification processor 120 quantifies the subjective fatigue and objective fatigue felt by a subject, based on the physiological amount acquired by physiological amount acquirer 110 (S2 in FIG. 2A).

Then, mode acquirer 320 acquires desired effects of intervention control that mode selector 310 prompts a user to select (S3 in FIG. 2A). When presenting to the user, mode selector 310 prepares the desired effects of intervention control in advance as options. Examples of the options include "Intervention Effect Mode" that prioritizes the intervention effects on fatigue, and "Work-Efficiency Mode" that prioritizes the work efficiency. Other than "Intervention Effect Mode" and "Work-Efficiency Mode", the options presented to the user may also be other modes such as "Fatigue Recovery Mode" and "Task Progress Mode" as long as their names prioritize either the intervention effects or the work efficiency.

Then, intervention controller 140 selects an intervention control scheme based on the mode selected by the user (S4 in FIG. 2A).

Intervention controller 140 references the relational data stored in first storage 130 to acquire the intervention effects and the work efficiency for each of multiple intervention control schemes and to select an intervention control scheme based on the mode selected by the user via mode acquirer 320. That is, if the user has selected the mode that prioritizes the intervention effects (selected "Intervention-effect priority mode" in S4 in FIG. 2A), an intervention control scheme with the highest intervention effects is selected from among the multiple intervention control schemes (S5a in FIG. 2A). If the user has selected the mode that prioritizes the work efficiency (selected "Work-efficiency priority mode" in S4 in FIG. 2A), an intervention control scheme with the highest work efficiency is selected from among the multiple intervention control schemes (S5b in FIG. 2A).

FIG. 2B is a flowchart showing a detailed operation performed in step S5a in FIG. 2A. When the user has selected the intervention-effect priority mode in step S4 of FIG. 2A, intervention controller 140 determines which fatigue is worse, the subjective fatigue or the objective fatigue quantified and acquired from quantification processor 120 (S21 in FIG. 2B).

If the value of the objective fatigue is greater than the value of the subjective fatigue (Yes in S21 in FIG. 2B), intervention controller 140 selects an intervention control scheme with the highest intervention effect against the objective fatigue from among the multiple intervention control schemes (S22a in FIG. 2B). If the value of the subjective fatigue is greater than the value of the objective fatigue or if the value of the objective fatigue and the value of the subjective fatigue value are equal (No in S21 in FIG. 2B), intervention controller 140 selects an intervention control scheme with the highest intervention effect on the subjective fatigue from among the multiple intervention control schemes (S22b in FIG. 2B).

For example, FIG. 8 shows one example of how the intervention control scheme is selected. As shown in FIG. 8, the degree of fatigue is presented on a seven-level equidistant scale, and the work efficiency is expressed as the number of input characters per minute. Using the equidistant scale allows the degrees of the subjective fatigue and the objective fatigue to be indicated by values within a predetermined number of levels. Here, the degrees of the subjective fatigue and the objective fatigue are indicated by the seven-level values. The higher the value, the higher the degree of fatigue. The degree of user A's subjective fatigue is at Level 4, and the degree of user A's objective fatigue is at Level 7. That is, the objective fatigue is greater than the subjective fatigue ((a) in Fig. 8). Therefore, if user A has selected the intervention-effect priority mode, an intervention control scheme using a wind with the highest intervention effect on the objective fatigue is selected. If user A has selected the work-efficiency priority mode, an intervention control scheme using a scent is selected ((b) in FIG. 8). The degree of user B's subjective fatigue is at Level 7, and the degree of user B's objective fatigue is at Level 4. That is, the subjective fatigue is greater than the objective fatigue ((c) in FIG. 8). Therefore, if user B has selected the intervention-effect priority mode, an intervention control scheme using a sound with the highest intervention effect on the subjective fatigue is selected. If user B has selected the work-efficiency priority mode, an intervention control scheme using a scent is selected ((d) in FIG. 8).

In the present embodiment, when the value of the subjective fatigue and the value of the objective fatigue are equal, an intervention control scheme with the highest intervention effect on the subjective fatigue is selected, but it is also possible to select an intervention control scheme with the highest intervention effect on the objective fatigue.

Intervention controller 140 controls intervention equipment 150 in accordance with the selected intervention control scheme (S6 in FIG. 2A).

Although mind-body state intervention apparatus 100 according to Embodiment 1 includes mode acquirer 320 and acquires the result of selection by mode selector 310, i.e., the result of prompting the user to select either the mode that prioritizes the intervention effects or the mode that prioritizes the work efficiency, mode acquirer 320 may not always be included. Moreover, although the intervention control scheme is selected depending on the mode selected by the user, the mode may not always be necessary. Instead, intervention control may always be performed to prioritize the intervention effects, or intervention control may always be performed to prioritize the work efficiency. In this case, the relational data only needs to indicate the relationships between each of the multiple intervention control schemes and whichever one is needed: the intervention effects or the work efficiency.

Although the above description has used fatigue as one example of the mind-body state, any other state such as sleepiness or anger may be used as the mind-body state.

For sleepiness, physiological amount detector 160 is, for example, a camera mounted on a personal computer. The physiological amount is acquired from a face image of a person captured by the camera. Examples of the physiological amount include the blink rate, the open-eye rate, and the pupil diameter. The methods of acquiring the blink rate and the pupil diameter have already been described above, and therefore redundant description is omitted here. The open-eye rate refers to the ratio of time during which the degree of eye opening per minute is 20% or less when the degree of eye opening during awakening is assumed to be 100%.

Other than a camera, the physiological amount detector may also be a heart rate sensor or an electroencephalograph.

In the case of using a heart rate sensor, examples of the physiological amount include LF and HF of the heart rate variability, the heart rate, or the respiration rate acquired from the heart rate sensor. The methods of acquiring LF and HF, the heart rate, and the respiration rate have already been described above, and therefore redundant description is omitted here.

In the case of using an electroencephalograph, the physiological amount is, for example, reactive α-wave band power to be acquired. The reactive α-wave band power refers to the ratio of the α-wave band power (8 Hz to 13 Hz) to 0.5- to 50-Hz band power, obtained through fast Fourier transform of brain waves performed at predetermined time intervals. The α waves are generally known to be relevant to sleepiness and become dominant in the brain waves when the degree of sleepiness is high.

Quantification processor 120 quantifies the subjective sleepiness and the objective sleepiness by a quantification technique that is preset based on the physiological amount that physiological amount acquirer 110 has acquired from physiological amount detector 160.

Here, the technique for quantifying the subjective sleepiness and the objective sleepiness is described. One example of the quantification technique is using sleepiness functions. The sleepiness functions refer to functions that take the physiological amount as input and output the subjective sleepiness and the objective sleepiness that have been quantified. Here, sleepiness functions using the open-eye rate shown in FIG. 9 is described as one example. The functions of the subjective sleepiness and the objective sleepiness with respect to the open-eye rate are created in advance through experiment. Examples of the method of acquiring the subjective sleepiness include VAS, the Karolinska sleepiness scale, and the Stanford sleepiness scale, and acquired values are defined as quantified values. The method of acquiring the objective sleepiness is, for example, acquiring the physiological amount. For example, the objective sleepiness is quantified from the reactive α-wave band power obtained by brain-wave measurement, the concentration of oxygenated hemoglobin obtained by functional near-infrared spectroscopy (fNIRS), or skin temperature measured at a predetermined site by thermography. Other than the technique using the physiological amount, it is also possible to use a technique for allowing an experimenter to objectively assess the sleepiness level of a subject from the face image of the subject.

As one example, FIG. 9 shows the sleepiness functions when the degrees of the subjective sleepiness and the objective sleepiness are quantified as values on a nine-level equidistant scale based on the Karolinska sleepiness scale and the open-eye rate is used as the physiological amount. The degrees of sleepiness are indicated as follows: Level 1, completely awake; Level 3, awake; Level 5, none of these; Level 7, sleepy; and Level 9, very sleepy. The subjective sleepiness and the objective sleepiness can be quantified by inputting the open-eye rate acquired by the physiological amount detector as the input value for the sleepiness functions. The subjective sleepiness can be quantified through self-declaration, whereas the objective sleepiness can be quantified by, for example, objective evaluation of facial expressions using nine levels of the Karolinska sleepiness scale. Although the above has described, as one example, the method using the sleepiness functions when the degrees of the subjective sleepiness and the objective sleepiness are quantified as values expressed on the nine-level equidistant scale and the open-eye rate is used as the physiological amount, both the method of acquiring the subjective sleepiness and the objective sleepiness and the physiological amount that is used as the input value are not limited to the above example.

Other than the technique using the sleepiness functions, the technique for quantifying the subjective sleepiness and the objective sleepiness may be using a physiological amount-sleepiness correspondence table or using machine learning. The technique for quantifying the subjective sleepiness and the objective sleepiness is not limited to using the sleepiness functions. In the case of using a physiological amount-sleepiness correspondence table, subjective sleepiness and objective sleepiness are quantified based on the magnitude of the physiological amount acquired by the physiological amount detector in accordance with a pre-set correspondence table between the physiological amount and each of the subjective sleepiness and the objective sleepiness. The physiological amount-sleepiness correspondence table differs from the physiological amount-fatigue correspondence table only in that the pre-set physiological amount is the open-eye rate, and all other aspects are identical. Thus, redundant description is omitted here.

In the case of using machine learning, one example is using a neural network. In the case of using a neural network, the neural network can be used in the same manner as in the case of fatigue. The quantification of fatigue using a neural network has already been described above and differs only in that the physiological amount acquired by the physiological amount detector and used as a feature. Thus, redundant description is omitted here.

Other than using a neural network, machine learning may also use multiple regression or random forest. The quantification of fatigue using multiple regression or random forest has already been described above and differs only in the physiological amount acquired by the physiological amount detector and used as a feature. Thus, redundant description is omitted here. The machine learning technique is not specified here. Moreover, the subjective sleepiness and the objective sleepiness may be quantified using different physiological amounts.

In the case where sleepiness is used as the mind-body state, first storage 130 stores relational data that indicates the relationships between each of multiple intervention control schemes and intervention effects on the subjective sleepiness and the objective sleepiness that have been quantified, and the relationships between each of the multiple intervention control schemes and work efficiency. The methods of calculating the intervention effects and the work efficiency and the method of creating the relational data are the same as those described in the case of fatigue, and therefore redundant description is omitted here.

Mode selector 310 prompts the user to input an item that the user wants to prioritize when selecting an intervention control scheme. Mode acquirer 320 acquires the mode selected by the user via mode selector 310. This operations are the same as those described in the case of fatigue, and therefore redundant description is omitted here.

Intervention controller 140 references the relational data stored in first storage 130 to select an intervention control scheme from among the multiple intervention control schemes, based on the intervention effects on the subjective sleepiness and the objective sleepiness that have been quantified, the work efficiency, and the mode acquired by mode acquirer 320. Intervention controller 140 controls intervention equipment 150 in accordance with the selected intervention control scheme. Intervention equipment 150 is, for example, an air conditioner. Upon receiving a signal from intervention controller 140, the air conditioner exposes people to a wind that is effective against sleepiness. The wind effective against sleepiness is generally a wind that causes the sympathetic nervous system to become dominant by exposure and that is characterized by having stimulant effects. Examples of the wind effective against sleepiness include a wind with strong fluctuations, a gentle wind that hits specific areas such as faces, and a wind with strong temperature fluctuations. Here, the wind effective against sleepiness is not specified. Other than an air conditioner, intervention equipment 150 may also be audio equipment or a vibration generator. Here, intervention equipment 150 is not specified.

In the case where the intervention equipment is audio equipment, a sound that is effective against sleepiness is produced. The sound effective against sleepiness is generally a sound that causes the sympathetic nervous system to become dominant by exposure and that is characterized by having stimulant effects. Examples of the sound effective against sleepiness include a sound that contains a large number of low-frequency components, a sound with high sound pressure, and a sound that contains dissonance. Here, the sound effective against sleepiness is not limited to the above examples.

In the case where the intervention equipment is a vibration generator, vibrations that are effective against sleepiness are transmitted to a person. The vibrations effective against sleepiness are generally vibrations that cause the sympathetic nervous system to become dominant when received and that are characterized by having stimulant effects. Examples of the vibrations effective against sleepiness include vibrations with high amplitudes, vibrations with high frequencies, and vibrations with irregular amplitudes. Here, the vibrations effective against sleepiness are not limited to the above examples.

In the case where anger is used as the mind-body state, physiological amount detector 160 is, for example, a camera mounted on a personal computer. The physiological amount is acquired from a face image of a person captured by the camera. Examples of the physiological amount include pulse waves, the pupil diameter, and the degree to which the corners of the mouth turn up. The method of acquiring the pupil diameter has already been described above, and therefore redundant description is omitted here. The pulse waves are acquired from time-varying waveforms of average luminance values of RGB by, for each moving image frame, calculating the average luminance values in a predetermined region of a face image captured by the camera. Through independent component analysis of the time-varying waveforms of the average luminance values, three independent waveforms are extracted, and among them, the one that contains a peak with the highest power spectrum is used to acquire the pulse waves. The degree to which the corners of the mouth turn up is acquired by setting nodes in a face image and obtaining the amounts of change in the coordinates of nodes corresponding to the corners of the mouth.

Other than a camera, for example, the physiological amount detector may be a heart rate sensor or an electrodermal activity meter. That is, the physiological amount detector is not limited to a camera.

In the case of using a heart rate sensor, examples of the physiological amount include LF and HF of the heart rate variability, the heart rate, and the respiration rate acquired by the heart rate sensor. The methods of acquiring LF and HF, the heart rate, and the respiration rate have already been described above, and therefore redundant description is omitted here.

In the case of using an electrodermal activity meter, the physiological amount is, for example, an electrodermal activity that is acquired. The electrodermal activity is said to be related to a sweating phenomenon, and it generally increases in an anger state.

Quantification processor 120 quantifies the subjective anger and the objective anger by a quantization technique that is preset based on the physiological amount acquired by the physiological amount acquirer via physiological amount detector 160.

A technique for quantifying the subjective anger and the objective anger is described here. One example of the quantification technique is using anger functions. The anger functions refer to functions that take the physiological amount as input and outputs the subjective anger and the objective anger that have been quantified. Here, the anger functions using the pupil diameter shown in FIG. 10 are described by way of example. The functions of the subjective anger and the objective anger with respect to the pupil diameter are created in advance through experiment. Examples of the method of acquiring the subjective anger include VAS, NRS, and the FaCE scale, in which acquired values are defined as quantified values. The method of acquiring the objective anger is acquiring the physiological amount. For example, the objective anger is quantified from reactive α-wave band power obtained by brain-wave measurement, the concentration of oxygenated hemoglobin obtained by fNIRS, or the saliva volume.

As one example, FIG. 10 shows the anger functions when the degrees of the subjective anger and the objective anger are quantified as values expressed on a seven-level equidistant scale and the pupil diameter is used as the physiological amount. Here, the higher the level value, the greater the degree of anger. For example, Level 1 represents a calm state, and Level 7 represents the most intense anger state. Although the degree of anger has been described using the above as one example, the way of expressing the anger level is not limited to the above example. The subjective anger and the objective anger can be quantified by inputting the pupil diameter acquired by the physiological amount detector as the input value for the anger functions. Although one example has been described above for the method using the anger functions when the subjective anger and the objective anger are quantified on the seven-level equidistant scale and the pupil diameter is used as the physiological amount, the above description does not intend to limit the method of acquiring the subjective anger and the objective anger and the physiological amount used as the input value.

In the case of using a physiological amount-anger correspondence table, the subjective anger and the objective anger are quantified from the magnitude of the physiological amount acquired by the physiological amount detector in accordance with a preset correspondence table between the physiological amount and the subjective and objective anger. The physiological amount-anger correspondence table and the physiological amount-fatigue correspondence table differ only in the pre-set physiological amount but are otherwise the same, and therefore redundant description is omitted here. The methods of acquiring the subjective anger and the objective anger are the same as those described as one example in the case of the anger functions, and therefore redundant description is omitted here.

In the case of using machine learning, one example is using a neural network. In the case of using a neural network, the neural network can be used in the same manner as in the case of fatigue. The quantification of fatigue using a neural network has already been described above, and the quantization of anger differs only in the physiological amount acquired as a feature by the physiological amount detector and is otherwise the same. Thus, redundant description is omitted here.

Other than using a neural network, machine learning may also use multiple regression or random forest. The quantification of fatigue using multiple regression or random forest has already been described above, and the quantization of anger differs only in the physiological amount acquired as a feature by the physiological amount detector and is otherwise the same. Thus, redundant description is omitted here. Here, the technique for machine learning is not specified. Alternatively, the subjective anger and the objective anger may be quantified using different physiological amounts.

In the case where anger is used as the mind-body state, first storage 130 stores relational data that indicates the relationships between each of multiple intervention control schemes and intervention effects on the subjective anger and the objective anger that have been quantified, and the relationships between each of the multiple intervention control schemes and work efficiency. The methods of calculating the intervention effects and the work efficiency and the method of creating the relational data are the same as those described as one example in the case of fatigue, and therefore redundant description is omitted here.

Mode selector 310 prompts the user to input an item that the user wants to prioritize when selecting an intervention control scheme. Mode acquirer 320 acquires the mode selected by the user via mode selector 310. These operations are the same those described as one example in the case of fatigue, and therefore redundant description is omitted here.

Intervention controller 140 references the relational data stored in first storage 130 to select an appropriate intervention control scheme from among multiple intervention control schemes, based on the intervention effects on the subjective anger and the objective anger that have been quantified, the work efficiency, and the mode selected by mode acquirer 320.

Intervention controller 140 controls intervention equipment 150 in accordance with the selected intervention control scheme. Intervention equipment 150 is, for example, an air conditioner. Upon receiving a signal from intervention controller 140, the air conditioner exposes a person to a wind that is effective against anger. The wind effective against anger is generally a wind that causes the parasympathetic nervous system to become dominant by exposure and that is characterized by having sedative effects. Examples of the wind effective against anger include a wind with 1/f fluctuations, a wind with low velocity and gentle fluctuations, and a wind with temperature fluctuations. The wind effective against anger is not specified here. Other than an air blower, intervention equipment 150 may also be audio equipment or a scent diffuser. Here, intervention equipment 150 is not specified.

In the case of using audio equipment, a sound that is effective against anger is produced. The sound effective against anger is generally a sound that causes the parasympathetic nervous system to become dominant by exposure and that is characterized by having sedative effects. Examples of the sound effective against anger include a sound with 1/f fluctuations, a sound with a gentle change in sound pressure, and bird chirping sounds. Here, the sound effective against anger is not specified.

In the case of using a scent diffuser, a person is exposed to a scent that is effective against anger. The scent effective against anger is generally a scent that causes the parasympathetic nervous system to become dominant by exposure and that is characterized by having sedative effects. Examples of the scent include a lavender scent, a bergamot scent, and a rose otto scent. Here, the scent effective against anger is not specified.

### [Advantageous Effects]

As described above, the intervention control based on the effect mode that the user wants to prioritize in intervention control brings about the following advantageous effects.

In general, intervention control for fatigue is performed according to the degree or type of fatigue, i.e., the degrees of the subjective fatigue and the objective fatigue. However, there is a problem that the individual's work status cannot be taken into account. For example, a system that performs intervention control by halting a use's operation even if the user has a lot of work to do is difficult for users to employ.

As in the present embodiment, the apparatus that allows a user to select either intervention control that prioritizes work efficiency or intervention control that prioritizes intervention effects brings about the effect of enabling the user to select a mode that prioritizes work efficiency when having a lot of work to do and to select a mode that prioritizes intervention effects when excessively tired. That is, it is possible to perform intervention effective for the user's needs.

Similarly, for sleepiness, the apparatus that allows a user to select either intervention control that prioritizes work efficiency or intervention control that prioritizes intervention effects brings about the effect of enabling the user to select a mode that prioritizes work efficiency when having a lot of work to do and to select a mode that prioritizes intervention effects when excessively sleepy. That is, it is possible to perform intervention effective for the user's needs.

Similarly, for anger, the apparatus that allows a user to select either intervention control that prioritizes work efficiency or intervention control that prioritizes intervention effects brings about the effect of enabling the user to select a mode that prioritizes work efficiency when having a lot of work to do and to select a mode that prioritizes intervention effects when extremely angry. That is, it is possible to perform intervention effective for the user's needs.

### [Summary]

As described above, mind-body state intervention apparatus 100 according to the present embodiment includes: physiological amount acquirer 110 that acquires a physiological amount of a subject detected by physiological amount detector 160; quantification processor 120 that quantifies two mind-body states based on the physiological amount, the two mind-body states including a mind-body state that the subject is aware of and a mind-body state the subject is unaware of; first storage 130 that stores relational data in advance, the relational data indicating the relationships between each of multiple intervention control schemes and intervention effects on the two mind-body states that have been quantified; and intervention controller 140 that performs intervention control with reference to the relational data, in which one intervention control scheme is selected from among the multiple intervention control schemes, based on the intervention effect on at least one of the two mind-body states quantified by quantification processor 120, and intervention equipment 150 is controlled in accordance with the one intervention control scheme selected.

This enables distinguishing between the mind-body state that the subject is aware of and the mind-body state that the subject is unaware of, and selecting an intervention control scheme based on this distinction. Accordingly, it is possible to select a more efficient and effect mind-body state intervention control scheme.

The mind-body state intervention apparatus further includes mode acquirer 320 that acquires a mode from mode selector 310 that prompts the subject to select either a mode that prioritizes intervention effects or a mode that prioritizes work efficiency, the work efficiency being the efficiency of work performed by the subject. The relational data further indicates the relationships between each of the multiple intervention control schemes and work efficiency. Intervention controller 140 selects an intervention control scheme based on the intervention effects, the work efficiency, and the mode.

This enables the subject to select either intervention that prioritizes the intervention effects on the mind-body states of the subject or intervention that prioritizes the workload of the subject. Accordingly, it is possible to select a mind-body state intervention control scheme that more reflects the preference of the subject.

The mind-body state intervention apparatus further includes a relational data creation device that creates the relational data from multiple subjects as targets and stores the relational data in first storage 130.

This enables newly creating and updating the relational data that serves as reference data in selecting an intervention control scheme.

The relational data creation device further includes workload acquirer 21 that acquires the volume of workload to be handled by the subject, detected by workload detector 23; work efficiency calculator 22 that calculates work efficiency based on the volume of workload; and intervention effect calculator 17 that calculates an intervention effect. The relational data creation device stores the work efficiency and the intervention effects that have been calculated, as the relational data in first storage 130.

This enables acquiring information necessary for the creation of the relational data that is used in selecting an intervention control scheme.

In the case where mode selector 310 has selected a mode that prioritizes an improvement of the intervention effects, intervention controller 140 references the relational data to select an intervention control scheme that yields the highest intervention effects, from among the multiple intervention control schemes.

Therefore, in the case where the subject has a request to prioritize intervention effects, it is possible to provide an intervention control scheme that reflects the request of the subject and yields the highest intervention effects.

In the case where mode selector 310 has selected a mode that prioritizes an improvement in work efficiency, intervention controller 140 references the relational data to select an intervention control scheme that yields highest work efficiency, from among the multiple intervention control schemes.

Therefore, in the case where the subject has a request to prioritize work efficiency, it is possible to provide an intervention control scheme that reflects the request of the subject and achieves highest work efficiency.

The intervention effects refers to the amounts of change in the mind-body states of the subject before and after intervention control.

Therefore, it is possible to select an intervention control scheme that focuses on changes in the mind-body states of the subject before and after intervention control.

The work efficiency refers to the rate of change in workload per unit time before and after intervention control of the subject, the unit time being a time required for the intervention control.

Therefore, it is possible to select an intervention control scheme that focuses on the rate of change in workload per unit time before and after intervention control of the subject, the unit time being a time required for the intervention control.

Hereinafter, further variations are described using fatigue as one example of the mind-body state.

### [Variation 1 of Embodiment 1]

Mind-body state intervention apparatus 200 according to Variation 1 of Embodiment 1 is configured to present, to a user, the subjective fatigue and the objective fatigue before and after intervention.

FIG. 11 is a block diagram showing a configuration of mind-body state intervention apparatus 200 according to Variation 1 of Embodiment 1. Mind-body state intervention apparatus 200 further includes second storage 410, amount-of-change calculator 420, and display 430, in addition to the configuration of mind-body state intervention apparatus 100.

In the present variation, physiological amount acquirer 110 and quantification processor 120 operate again as described above even after the intervention control in order to quantify the subjective fatigue and objective fatigue felt by the subject after the intervention control.

Second storage 410 stores the subjective fatigue and the objective fatigue that have been quantified before and after the intervention control and transmitted from quantification processor 120. Second storage 410 is, for example, read-only memory (ROM), random access memory (RAM), a hard disk drive (HDD), or a solid-state drive (SSD). Here, the storage is not specified.

Amount-of-change calculator 420 reads the subjective fatigue and the objective fatigue before and after the intervention control from second storage 410 and calculates the amounts of changes in the subjective fatigue and the objective fatigue. For example, in the case where the objective fatigue before the intervention control is at Level 5 and the objective fatigue after the intervention control is at Level 2, the amount of change in the objective fatigue is -3. Amount-of-change calculator 420 is realized by a program and a processor that executes the program.

Display 430 acquires the amounts of change in the subjective fatigue and the objective fatigue before and after the intervention control from amount-of-change calculator 420 and displays the acquired amounts of change to the user as shown in FIG. 12. FIG. 12 shows one example and does not intend to limit the way of displaying the amounts of change in the subjective fatigue and the objective fatigue before and after the intervention control. Display 430 is, for example, a display monitor of a personal computer.

### [Advantageous Effects]

As described above, presenting the subjective fatigue and the objective fatigue before and after intervention to the user brings about the following advantageous effects.

In general, intervention control on fatigue involves presenting the degree of and the intervention effect on only either of the subjective fatigue and the objective fatigue. The problem here is that the degree of or the intervention effect on only one of the subjective fatigue and the objective fatigue are presented to the user, and as a result the user may be unable to correctly recognize his/her own fatigue state and the intervention effects.

As in the present embodiment, the apparatus that presents, to the user, both the subjective fatigue and the objective fatigue before and after intervention brings about the effect of enabling the user to recognize his/her own fatigue state with higher accuracy and to recognize the intervention effects on fatigue with higher accuracy.

Similarly, for sleepiness, the subjective sleepiness and the objective sleepiness before and after intervention are presented to the user. This enables the user to recognize his/her own sleepiness state with higher accuracy and to recognize the intervention effects on sleepiness with higher accuracy.

Similarly, for anger, the subjective anger and the objective anger before and after intervention are presented to the user. This enables the user to recognize his/her own anger state with higher accuracy and to recognize the intervention effect on anger with higher accuracy.

Although second storage 410 and amount-of-change calculator 420 are additionally provided as means for presenting fatigue before and after intervention to the user, the means for presenting fatigue before and after intervention to the user is of course not limited to this example. The configuration of the means is not limited as long as it has similar functions.

As described above, intervention controller 140 according to the present embodiment presents, to the subject, the amounts of change in the mind-body state before and after intervention control.

This enables the subject to recognize the amounts of change in his/her mind-body state before and after intervention control. Accordingly, the subject is able to both experience the intervention effects and become aware of his/her own mind-body state.

### [Variation 2 of Embodiment 1]

Mind-body state intervention apparatus 300 according to Variation 2 of Embodiment 1 is configured to present an intervention control scheme to a user, in addition to the subjective fatigue and the objective fatigue before and after intervention.

FIG. 13 is a block diagram showing a configuration of mind-body state intervention apparatus 300 according to Variation 2 of Embodiment 1. Mind-body state intervention apparatus 300 further includes second storage 410, amount-of-change calculator 420, and display 430, in addition to the configuration of mind-body state intervention apparatus 100.

In the present variation, physiological amount acquirer 110 and quantification processor 120 operate again as described above even after intervention control in order to quantify the subjective fatigue and objective fatigue felt by the subject after intervention control.

Unlike in Variation 1 of Embodiment 1, second storage 410 stores not only the subjective fatigue and the objective fatigue that have been quantified before and after intervention control and transmitted from quantification processor 120 , but also the intervention control scheme transmitted from intervention controller 140.

Amount-of-change calculator 420 calculates the amounts of change in the subjective fatigue and the objective fatigue before and after intervention control, which are read from second storage 410, as in Variation 1 of Embodiment 1. For example, in the case where the objective fatigue before intervention control is at Level 5 and the objective fatigue after the intervention control is at Level 2, the amount of change in the objective fatigue is -3.

Display 430 acquires the amounts of changes in the subjective fatigue and the objective fatigue before and after intervention control from amount-of-change calculator 420, and displays the acquired amounts of change to the user, together with the intervention control scheme stored in second storage 410, as shown in FIG. 14. FIG. 14 shows one example and does not intend to limit the way of displaying the amounts of changes in the subjective fatigue and the objective fatigue before and after intervention control, or the intervention control scheme that has been performed.

### [Advantageous Effects]

As described above, presenting the intervention control scheme in addition to the subjective fatigue and the objective fatigue before and after intervention to the user brings about the following advantageous effects.

In general, intervention control on fatigue involves presenting the degree of or the intervention effect on only either of the subjective fatigue and the objective fatigue. The problem here is that the user may be unable to recognize what intervention control scheme was used on the user and may cast doubt on the intervention control.

As in the present embodiment, if not only the subjective fatigue and the objective fatigue before and after intervention but also the intervention control scheme is presented to the user, the user is able to recognize the intervention control scheme used on the user and the intervention effects achieved thereby. This brings about the effect of dismissing doubts about the intervention control.

Similarly, for sleepiness, presenting not only the subjective sleepiness and the objective sleepiness before and after intervention but also the intervention control scheme to the user enables the user to recognize the intervention control scheme used on the user and the intervention effects achieved thereby. This brings about the effect of dismissing doubts about the intervention control.

Similarly, for anger, presenting not only the subjective anger and the objective anger before and after intervention but also the intervention control scheme to the user enables the user to recognize the intervention control scheme used on the user and the intervention effects achieved thereby. This brings about the effect of dismissing doubts about the intervention control.

Although second storage 410 and amount-of-change calculator 420 are additionally provided as means for presenting the intervention control scheme to the user, the means for presenting the intervention control scheme to the user is of course not limited to this example. The configuration of the means is not specified as long as it has similar functions.

As described above, intervention controller 140 according to the present embodiment presents the intervention control scheme used in intervention control to the subject.

This enables the subject to recognize the intervention control scheme and the effects achieved thereby. Thus, it is possible to dismiss the subject's doubts about the intervention control.

### [Embodiment 2]

### [Configuration]

FIG. 15 is a block diagram showing a characteristic functional configuration of mind-body state intervention apparatus 400 according to Embodiment 2. Mind-body state intervention apparatus 400 has a function of changing the intervention control scheme that has been used until then when the intervention effects fall below a predetermined threshold value. Mind-body state intervention apparatus 400 differs from mind-body state intervention apparatus 100 according to Embodiment 1, in that second storage 410, amount-of-change calculator 420, and intervention effect evaluator 440 are additionally provided. Focusing on this point, operations and effects are described hereinafter. The description here uses fatigue as one example of the mind-body state.

Second storage 410 stores the subjective fatigue and the objective fatigue quantified before and after intervention control and transmitted from quantification processor 120, in association with the date and time when the intervention control has been performed. The configuration of second storage 410 has already been described in Variation 1 of Embodiment 1, and therefore redundant description is omitted here.

Amount-of-change calculator 420 reads the subjective fatigue, the objective fatigue, and the date and time from second storage 410 and, based on this data, calculates the amounts of change in the subjective fatigue and the objective fatigue within a predetermined of time. That is, the amount-of-change calculator calculates the amounts of change between the subjective fatigue and the objective fatigue at the start of intervention control and the subjective fatigue and the objective fatigue after a predetermined period of time has elapsed.

The calculation here requires the subjective fatigue and the objective fatigue quantified at a point in time when the predetermined period of time has elapsed. Thus, in the present embodiment, physiological amount detector 160, physiological amount acquirer 110, and quantification processor 120 repeat a series of operations for quantifying the physiological amount of the subject (S1 and S2 in FIG. 2A) at regular time intervals necessary and sufficient to perform the series of operations.

Intervention effect evaluator 440 compares the magnitude of the amount of change in each of the subjective fatigue and the objective fatigue acquired within a predetermined period of time from amount-of-change calculator 420 with a preset threshold value to determine which is greater. In the case where the amounts of change in both of the subjective fatigue and the objective fatigue fall below the threshold value, intervention controller 140 changes the current intervention control. Intervention effect evaluator 440 is realized by a program and a processor that executes the program.

### [Operations]

FIG. 16 is a flowchart for explaining operations of the mind-body state intervention apparatus 400 according to Embodiment 2. The operations of mind-body state intervention apparatus 400 are the same as those of mind-body state intervention apparatus 100 up to a certain point. Specifically, operations performed in step S6 and subsequent steps in FIG. 2A are different. Therefore, step S6 and subsequent steps in FIG. 2A are described in detail with reference to FIG. 16.

First, intervention controller 140 checks the mode acquired by the mode acquirer when selecting the currently active intervention control scheme (step S31 in FIG. 16). Subsequent operations vary depending on the mode checked by intervention controller 140 (step S32 in FIG. 16). In the case where the mode is the work-efficiency priority mode (the work-efficiency priority mode is selected in step S32 in FIG. 16), intervention controller 140 continues to use the current intervention control scheme (step S35b in FIG. 16). In the case where the mode is the intervention-effect priority mode (the intervention-effect priority mode is selected in step S32 in FIG. 16), intervention effect evaluator 440 determines the intervention effect by comparing the magnitudes between the predetermined threshold value and the amount of change in each of the subjective fatigue and the objective fatigue within a predetermined period of time (step S33 in FIG. 16).

If the intervention effect is higher than or equal to the threshold value, i.e., if there is the intervention effect higher than or equal to a reference value (No in S34 in FIG. 16), intervention controller 140 continues to use the current intervention control scheme (S35b in FIG. 16).

If the intervention effect is lower than the threshold value, i.e., the intervention effect falls below the reference value (Yes in S34 in FIG. 16), intervention controller 140 changes the intervention control scheme. At this time, an intervention control scheme with the highest intervention effects is selected, excluding the current intervention control scheme (S35a in FIG. 16).

In the present embodiment, the determination of the intervention effects is based on whether the intervention effects are higher than or equal to the threshold value or lower than the threshold value, but this determination may also be based on whether the intervention effects are higher than the threshold value or lower than or equal to the threshold value.

For example, FIG. 17 shows one example of changing the intervention control scheme according to Embodiment 2. As shown in FIG. 17, the degrees of user C's fatigue are as follows: the degree of the subjective fatigue is at Level 4 and the degree of the objective fatigue is at Level 7, i.e., the objective fatigue is greater ((a) in FIG. 17). In the case where the intervention-effect priority mode has been selected, an intervention control scheme using a wind that has the highest intervention effect on the objective fatigue is selected ((c) in FIG. 17). However, the intervention effect decreases over time due to habituation ((b) in Fig. 17). If the intervention effect falls below the threshold value, then an intervention control scheme using a scent that has the second highest intervention effect on the objective fatigue is selected ((c) in Fig. 17).

### [Advantageous Effects]

As described above, changing the intervention control scheme when the amounts of change in the subjective mind-body state and the objective mind-body state fall below the threshold value brings about the following advantageous effects.

In general, intervention control on fatigue does not produce constant intervention effects, but produces time-varying intervention effects. For example, in the case of intervention using a scent, even if high intervention effects are obtained at the start of intervention control, habituation over time reduces the intervention effects. Therefore, if the intervention time exceeds a certain period of time, the intervention effects become virtually nonexistent, rendering the intervention ineffective.

As in the present embodiment, if the intervention control scheme is changed when the amounts of change in the subjective fatigue and the objective fatigue fall below the threshold value, it is possible to suppress a reduction in the intervention effects due to habituation and to achieve effective intervention control.

Moreover, there are individual differences in the magnitudes of the intervention effects on fatigue by intervention control and in variations of the intervention effects over time. For example, in the case of intervention using temperature, the intervention effects on women are significant but diminish significantly over time, whereas the intervention effects on men are small but diminishes little over time. That is, there is a problem that even if intervention control is performed for the same amount of time, it may be effective for women but not for men.

As in the present embodiment, if the intervention control scheme is changed when the amounts of change in the subjective fatigue and the objective fatigue fall below the threshold value, it is possible to suppress a reduction in the intervention effects due to individual differences. For example, in the case of intervention using temperature, effective intervention control may be realized by shortening the temperature-based intervention control time for women while lengthening the temperature-based intervention control time for men before switching to the next intervention control scheme.

Similarly, for sleepiness, changing the intervention control scheme when the amounts of change in the subjective sleepiness and the objective sleepiness fall below the threshold value brings about the effect of suppressing a reduction in the intervention effects due to individual differences.

Similarly, for anger, changing the intervention control scheme when the amounts of change in the subjective anger and the objective anger fall below the threshold value brings about the effect of suppressing a reduction in the intervention effects due to individual differences.

Although second storage 410, amount-of-change calculator 420, and intervention effect evaluator 440 are additionally provided here as means for changing the intervention control means, the means for causing the user to change the intervention control means is not limited to this example. The configuration of the means is not limited as long as it has similar functions.

### [Variation 1 of Embodiment 2]

Variation 1 of Embodiment 2 involves gradually increasing the intensity of intervention control performed using specific intervention equipment over a certain period of time.

Variation 1 has the same configuration as mind-body state intervention apparatus 400 according to Embodiment 2, but differs in the operations of intervention controller 140. The following description focuses on this point.

Intervention controller 140 references the relational data stored in storage 130 to select an intervention control scheme based on the mode acquired by mode acquirer 320. At this time, intervention equipment 150 is controlled to perform intervention in stages. That is, when controlling intervention equipment 150 in accordance with the selected intervention control scheme, intervention controller 140 performs control so that the intensity of intervention increases over time.

For example, to describe a scent as one example of the intervention control scheme as shown in FIG. 18, intervention control is performed with low scent intensity and low intervention effects for the first 10 minutes, and then performed with normal scent intensity and medium intervention effects for the next 20 minutes. Then, intervention control is performed with high scent intensity and high intervention effects for the next 30 minutes.

Although a scent is used as one example, the intervention control scheme may also be a sound or a wind. The stepwise intervention control scheme is not limited to the above example. In the case of using a sound, intervention control may involve increasing sound fluctuations at regular intervals, increasing the sound volume, or raising the sound frequency. In the case of using wind, intervention control may involve increasing the wind intensity, increasing wind fluctuations, or changing the volume of wind that blows against a person.

### [Advantageous Effects]

As described above, performing intervention control stepwise on the subjective mind-body state and the objective mind-body state over a certain period of time brings about the following advantageous effects.

In general, intervention control on fatigue does not produce constant intervention effects, but produces time-varying intervention effects. For example, in the case of intervention using a scent, even if high intervention effects are obtained at the start of intervention control, habituation over time reduces the intervention effects. Therefore, if the intervention time exceeds a certain period of time, the intervention effects become virtually nonexistent, rendering the intervention ineffective.

By performing intervention control stepwise as in the present embodiment, it is possible to suppress a reduction in the intervention effects due to habituation and to achieve effective intervention control.

Similarly, for sleepiness, performing intervention control stepwise suppresses a reduction in the intervention effects due to habituation, thereby rendering intervention control effective.

Similarly, for anger, performing intervention control stepwise suppresses a reduction in the intervention effects due to habituation, thereby rendering intervention control effective.

Although second storage 410, amount-of-change calculator 420, and intervention effect evaluator 440 are additionally provided as means for changing the intervention control means, the means for causing the user to change the intervention control means is not limited to this example. The configuration of the means is not limited as long as it has similar functions.

### [Embodiment 3]

### [Configuration]

FIG. 19 is a block diagram showing a characteristic functional configuration of mind-body state intervention apparatus 500 according to Embodiment 3.

Mind-body state intervention apparatus 500 has a function of selecting an intervention control scheme suitable for an individual subject. Mind-body state intervention apparatus 500 differs from mind-body state intervention apparatus 100 according to Embodiment 1 in that second storage 410, amount-of-change calculator 420, and optimization processor 450 are additionally provided. The following description focuses on this point. The description here uses fatigue as one example of the mind-body state.

Second storage 410 stores the subjective fatigue and the objective fatigue quantified and transmitted from quantification processor 120, the date and time, the amounts of change in the subjective fatigue and the objective fatigue within a predetermined period of time, transmitted from amount-of-change calculator 420, and the intervention control scheme transmitted from intervention controller 140.

Amount-of-change calculator 420 is the same as amount-of-change calculator 420 described in Embodiment 2, and therefore redundant description is omitted here.

Optimization processor 450 selects an optimal intervention control scheme for an individual, based on the subjective fatigue, the objective fatigue, and the date and time that have been read from second storage 410, the amounts of change in the subjective fatigue and the objective fatigue within a predetermined period of time, and the intervention control scheme. Optimization processor 450 is realized by a program and a processor that executes the program.

An optimization technique is, for example, using a neural network. In the case of using a neural network, as shown in FIG. 20, at least one of the subjective fatigue, the objective fatigue, the date and time, and the amounts of change in the subjective fatigue and the objective fatigue within a predetermined period of time is used as input, and the intervention control scheme is used as output. Although the use of a neural network is described as one example of the optimization technique, the optimization technique may also be using k-nearest neighbors or random forest. The optimization technique is not limited to the above example.

In the case of using k-nearest neighbors or random forest, as shown in FIGS. 21 and 22, at least one of the subjective fatigue, the objective fatigue, the date and time, and the amounts of change in the subjective fatigue and the objective fatigue within a predetermined period of time is used as input, and the intervention control scheme is used as output.

Intervention controller 140 references the relational data stored in storage 130 to select an intervention control scheme based on the mode acquired by mode acquirer 320 and the processing of optimization processor 450. For example, in the case where the user selects the intervention-effect priority mode, an intervention control scheme with the highest intervention effects is selected from among multiple intervention control schemes. However, if there is a signal received from optimization processor 450, the intervention control scheme selected by optimization processor 450 is prioritized. In the case where the user selects the work-efficiency mode, an intervention control scheme with the highest work efficiency is selected from among the multiple intervention control schemes.

Intervention equipment 150 is controlled based on the intervention control scheme selected by intervention controller 140. The control of intervention equipment 150 is the same as the control of intervention equipment 150 described in Embodiment 1, and therefore redundant description is omitted here.

### [Advantageous Effects]

As described above, the optimization for each individual based on the amounts of change in the subjective fatigue and the objective fatigue, the date and time, and the intervention control scheme brings about the following advantageous effects.

There are individual differences in the magnitudes of the intervention effects on fatigue by intervention control and in variations of the intervention effects over time. For example, in the case of intervention using temperature, the intervention effects on women are significant but diminish significantly over time, whereas the intervention effects on men are small but diminishes little over time. That is, there is a problem that even if intervention control is performed for the same amount of time, it may be effective for women but not for men.

As in the present embodiment, if the intervention control scheme is changed when the amounts of change in the subjective fatigue and the objective fatigue fall below the threshold value, it is possible to suppress a reduction in the intervention effects due to individual differences. For example, in the case of intervention using temperature, effective intervention control may be realized by shortening the temperature-based intervention control time for women while lengthening the temperature-based intervention control time for men before switching to the next intervention control scheme.

Similarly, for sleepiness, a reduction in the intervention effects due to individual differences can be suppressed by changing the intervention control scheme when the amounts of change in the subjective sleepiness and the objective sleepiness fall below the threshold value.

Similarly, for anger, a reduction in the intervention effects due to individual differences can be suppressed by changing the intervention control scheme when the amounts of change in the subjective anger and the objective anger fall below the threshold value.

Although second storage 410, amount-of-change calculator 420, and optimization processor 450 are additionally provided as means for suppressing a reduction in the intervention effects due to individual differences, the means for suppressing a reduction in the intervention effects due to individual differences is not limited to this example. The configuration of the means is not limited as long as it has similar functions.

### [Embodiment 4]

### [Configuration]

FIG. 23 is a block diagram showing a characteristic functional configuration of mind-body state intervention apparatus 600 according to Embodiment 4. Mind-body state intervention apparatus 600 has a function of creating a task schedule for a subject, based on the subjective fatigue and objective fatigue felt by the subject. Mind-body state intervention apparatus 600 differs from mind-body state intervention apparatus 100 according to Embodiment 1, in that schedule creator 510, operation terminal controller 520, and task terminal 530 are additionally provided. The following description focuses on this point. The description here uses fatigue as one example of the mind-body state.

Schedule creator 510 creates a user's schedule based on the subjective fatigue and the objective fatigue acquired from quantification processor 120. For example, in the case of creating a daily schedule, as shown in FIG. 24, if the user's subjective fatigue and objective fatigue are great at the start of work, the schedule is created so that the former half is to conduct work centered on simple tasks while avoiding tasks that require concentration, the middle part is to perform intervention control to reduce the subjective fatigue and the objective fatigue, and the latter half is to conduct work centered on tasks that require concentration. The schedule may be a monthly schedule or an annual schedule, and the time interval of the schedule is not limited to the above example.

Operation terminal controller 520 controls operation terminal 530 of the user in accordance with the schedule transmitted from schedule creator 510. For example, a notification such as "Intervention will begin" may be displayed on the screen of operation terminal 530 of the user at the start of intervention. Although a notification is used as one example, task terminal 530 may be powered off or a warning alarm may be sounded if the user is still working after the scheduled time. The control of task terminal 530 of the user is not limited to the above examples.

Schedule creator 510 and operation terminal controller 520 are realized by a program and a processor that executes the program.

Operation terminal 530 is a terminal where the subject actually performs tasks, and may, for example, be a personal computer or a smartphone.

Intervention controller 140 references the relational data stored in storage 130 to select an intervention control scheme based on the mode acquired by mode acquirer 320. At this time, intervention control is initiated and terminated based on the schedule acquired from schedule creator 510.

Intervention equipment 150 is controlled based on the intervention control scheme and the schedule selected by intervention controller 140. The control of intervention equipment 150 is the same as the control of intervention equipment 150 described in Embodiment 1, and therefore redundant description is omitted here.

### [Advantageous Effects]

As described above, creating a schedule based on the subjective fatigue and the objective fatigue and controlling the operation terminal of the user based on the schedule brings about the following advantageous effects.

In general, intervention control can reduce fatigue, but the fundamental issue is that the user is working under a schedule that is likely to cause fatigue. There is also a problem that, when the user performs tasks while his/her fatigue is accumulating, the concentration of the user declines and errors may occur.

As in the present embodiment, if the schedule is created based on the subjective fatigue and the objective fatigue, it is possible to avoid the accumulation of fatigue and the occurrence of operational errors.

Similarly, for sleepiness, the accumulation of sleepiness and the occurrence of operational errors can be avoided by creating the schedule based on the subjective sleepiness and the objective sleepiness.

Similarly, for anger, the accumulation of sleepiness and the occurrence of operational errors can be avoided by creating the schedule based on the subjective anger and the objective anger.

Although schedule creator 510, operation terminal controller 520, and task terminal 530 are additionally provided as means for performing intervention control based on the created schedule, the means for performing intervention control based on the created schedule is not limited to this example. The configuration of the means is not limited as long as it has similar functions.

### [Embodiment 5]

### [Configuration]

FIG. 25 is a block diagram showing a characteristic functional configuration of mind-body state intervention apparatus 700 according to Embodiment 5 of the present invention. Mind-body state intervention apparatus 700 has a function of analyzing a time zone in which the subjective fatigue and the objective fatigue are likely to accumulate. Mind-body state intervention apparatus 700 differs from mind-body state intervention apparatus 100 according to Embodiment 1, in that second storage 410 and control time analyzer 540 are additionally provided. The following description focuses on this point. The description here uses fatigue as one example of the mind-body state.

Second storage 410 stores the subjective fatigue and the objective fatigue felt by the subject before and after intervention control, quantified by quantification processor 120, in association with the date and time.

Control time analyzer 540 reads the subjective fatigue, the objective fatigue, and the date and time from second storage 410 and analyzes a time zone in which the subjective fatigue and the objective fatigue increase. For example, as shown in FIG. 26, average values of the subjective fatigue and the objective fatigue for each hour during weekly workdays are compared, and a time zone with the highest average value is derived. Intervention controller 140 performs intervention control so as to reduce the average value of the subjective fatigue and the objective fatigue within this highest time zone. In FIG. 26, the time zone with the highest average value of the subjective fatigue and the objective fatigue is from 17:00 to 18:00. Thus, an intervention control time is determined to further reduce the average value of the subjective fatigue and the objective fatigue within the time zone from 17:00 to 18:00.

Although the description uses the average value of the subjective fatigue and the objective fatigue for each hour as one example, an average value for each month or an average value for each season may also be used. The analysis technique is not limited to the above example. Control time analyzer 540 is realized by a program and a processor that executes the program.

Intervention controller 140 references the relational data stored in storage 130 to select an intervention control scheme based on the mode acquired by mode acquirer 320. At this time, intervention control is initiated and terminated based on the time zone of intervention control acquired from control time analyzer 540.

For example, in the case where intervention control is started at 16:00, an intervention control scheme is selected based on the intervention effects, the work efficiency, and the mode at 16:00. The method of selecting the intervention control scheme is the same as used in intervention controller 140 according to Embodiment 1, and therefore redundant description is omitted here.

Intervention equipment 150 is controlled based on the intervention control scheme and the time zone of intervention control selected by intervention controller 140. The control of intervention equipment 150 is the same as the control of intervention equipment 150 described in Embodiment 1, and therefore redundant description is omitted here.

### [Advantageous Effects]

As described above, analyzing the time zone in which the subjective fatigue and the objective fatigue are likely to accumulate and performing intervention control based on the analysis brings about the following advantageous effects.

In performing intervention control, there is an issue in which when the degree of user's fatigue is low, even if intervention control is performed, the intervention effects are low and the user may have difficulty to feel the intervention effects. As in the present embodiment, if intervention control is performed based on the time zone in which the subjective fatigue and the objective fatigue are likely to accumulate, intervention control can be performed to the degree of fatigue from becoming excessively high. This brings about the effect of enabling the user to easily feel the intervention effects.

Similarly, for sleepiness, the intervention control based on the time zone in which the subjective sleepiness and the objective sleepiness are likely to accumulate prevents the sleepiness level from becoming excessively high. This brings about the effect of enabling the user to easily feel the intervention effects.

Similarly, for anger, the intervention control based on the time zone in which the subjective anger and the objective anger are likely to accumulate prevents the anger level from becoming excessively high. This brings about the effect of enabling the user to easily feel the intervention effects.

Although second storage 410 and control time analyzer 540 are additionally provided as means for performing intervention control based on the relationship between the mind-body state and the time zone, the means for performing intervention control based on the relationship between the mind-body state and the time zone is not limited to the above example. The configuration of the means is not limited as long as it has similar functions.

### [Mind-Body State Intervention System]

FIG. 27 is an outside drawing of a mind-body state intervention system. The mind-body state intervention system includes a desktop personal computer that executes mind-body state intervention apparatus 100, physiological amount detector 160, intervention equipment 150 (150a to 150c), and mode selector 310. The desktop personal computer can control intervention equipment 150 via communication with intervention equipment 150.

Physiological amount detector 160 is realized by, for example, an in-camera provided in the desktop personal computer. Intervention equipment 150 is realized by, for example, air conditioner 150a, loudspeaker 150b, and aromatic diffuser 150c. Mode selector 310 is realized by, for example, a mouse, a keyboard, and a touch panel. The communication between intervention equipment 150 and mind-body state intervention apparatus 100 may be performed via cable communication or via wireless communication such as BLUETOOTH (registered trademark).

Note that mode selector 310 does not always be provided, and intervention equipment 150 may serve as any one or more of the devices listed above.

A relational-data creation system includes a desktop personal computer that executes relational data creation device 10, physiological amount detector 16, workload detector 23, and intervention equipment 150. The desktop personal computer can control intervention equipment 150 via communication with intervention equipment 150.

Physiological amount detector 16 and workload detector 23 are realized by, for example, an in-camera provided in the desktop personal computer. The communication between intervention equipment 150 and relational data creation device 10 may be performed via cable communication or via wireless communication such as Bluetooth.

### [Other Embodiments]

The mind-body state intervention apparatus, the mind-body state intervention system, and the mind-body state intervention method according to the present invention have been described thus far with reference to the embodiments. Note that the configurations described in the above embodiments are mere examples, and it goes without saying that various modifications can be made to the configurations without departing from the scope of the invention. It is also, of course, possible to use any combinations of each embodiment or variations of each embodiment.

For example, in the above-described embodiments, the mind-body state intervention system may be realized by one or a plurality of server devices. Thus, the term "system" as used in the specification of the present application may refer to a configuration using a single device or a configuration using multiple devices. In the case where the system is configured by multiple devices, constituent elements (particularly, functional constituent elements) provided in the system may be distributed in any way across the multiple devices.

The method of communication between devices in the above-described embodiments is not particularly limited. The communication between devices may be realized via a relay device (e.g., a broadband router), which is not shown.

In the above-described embodiments, processing executed by a specific processor may be executed by a different processor. It is also possible to change a sequence of multiple processing steps or to execute multiple processing steps in parallel.

In the above-described embodiments, each constituent element may be realized by executing a software program suitable for the constituent element. Each constituent element may also be realized by a program executor such as a CPU or a processor reading and executing a software program recorded on a recording medium such as a hard disk or semiconductor memory.

Each constituent element may also be realized as hardware. For example, each constituent element may also be a circuit (or an integrated circuit). These circuits may configure a single circuit as a whole, or may be different circuits. Each of these circuits may also be a general-purpose circuit or a dedicated circuit.

Note that general or specific aspects of the present invention may be realized as a system, a device, a method, an integrated circuit, a computer program, or a non-transitory recording medium such as a computer-readable CD-ROM. Alternatively, these aspects may also be realized as any combination of a system, a device, a method, an integrated circuit, a computer program, and a recording medium.

For example, the present invention may be realized as a method that is executed by a computer system such as an image information provision system or a face authentication system, or as a program for causing a computer system to execute the method. The present invention may also be realized as a non-transitory computer-readable recording medium that records such a program thereon.

The present invention also includes other modifications obtained by applying various changes conceivable by those skilled in the art to the embodiments or modifications, and other modifications realized by any combinations of constituent elements and functions described in the embodiments without departing from the scope of the present invention.

### [Appendices]

The features of the mind-body state intervention apparatus described based on the above embodiments are shown hereinafter.

### [Technique 1]

A mind-body state intervention apparatus includes: a physiological amount acquirer that acquires a physiological amount of a subject detected by a physiological amount detector; a quantification processor that quantifies, for a predetermined mind-body state, two mind-body states based on the physiological amount, the two mind-body states including a mind-body state that the subject is aware of and a mind-body state that the subject is unaware of; a storage that stores relational data in advance, the relational data indicating relationships between each of a plurality of intervention control schemes and intervention effects on the two mind-body states quantified; and an intervention controller that performs intervention control in which, with reference to the relational data, one intervention control scheme is selected from among the plurality of intervention control schemes, based on the intervention effect on at least one of the two mind-body states quantified by the quantification processor, and intervention equipment is controlled in accordance with the one intervention control scheme selected.

### [Technique 2]

The mind-body state intervention apparatus according to Technique 1 further includes a mode acquirer that acquires a mode from a mode selector, the mode selector prompting the subject to select either a mode that prioritizes an improvement in the intervention effects or a mode that prioritizes an improvement in work efficiency, the work efficiency being efficiency of work performed by the subject, wherein the relational data further indicates relationships between each of the plurality of intervention control schemes and the work efficiency, and the intervention controller selects an intervention control scheme based on the intervention effects, the work efficiency, and the mode.

### [Technique 3]

The mind-body state intervention apparatus according to Technique 1 or 2 further includes a relational data creation device that creates the relational data from a plurality of subjects as targets and stores the relational data in the storage.

### [Technique 4]

The mind-body state intervention apparatus according to Technique 3 is such that the relational data creation device includes: a workload acquirer that acquires a volume of work to be performed by the subject, detected by a workload detector; a work efficiency calculator that calculates work efficiency based on the volume of work; and an intervention effect calculator that calculates the intervention effects, and the relational data creation device stores the work efficiency calculated and the intervention effects calculated as the relational data in the storage.

### [Technique 5]

The mind-body state intervention apparatus according to any one of Techniques 2 to 4 is such that, when the mode selector selects a mode that prioritizes an improvement in the intervention effects, the intervention controller references the relational data to select an intervention control scheme that maximizes the intervention effects from among the plurality of intervention control schemes.

### [Technique 6]

The mind-body state intervention apparatus according to any one of Techniques 2 to 5 is such that, wherein, when the mode selector selects a mode that prioritizes an improvement in the work efficiency, the intervention controller references the relational data to select an intervention control scheme that maximizes the work efficiency from among the plurality of intervention control schemes.

### [Technique 7]

The mind-body state intervention apparatus according to any one of Techniques 1 to 6 is such that the intervention effects are amounts of change in a mind-body state before and after intervention control of the subject.

### [Technique 8]

The mind-body state intervention apparatus according to any one of Techniques 2 to 7 is such that the work efficiency is a rate of change in workload per unit time before and after intervention control of the subject, the unit time being a time required for the intervention control.

### [Technique 9]

The mind-body state intervention apparatus according to any one of Techniques 1 to 8 is such that the intervention controller presents, to the subject, an amount of change in the mind-body state before and after the intervention control.

### [Technique 10]

The mind-body state intervention apparatus according to any one of Techniques 1 to 9 is such that the intervention controller presents, to the subject, the intervention control scheme used in the intervention control.

### [Technique 11]

The mind-body state intervention apparatus according to any one of Techniques 1 to 10 is such that the mind-body state is fatigue felt by the subject.

### [Technique 12]

A mind-body state intervention system includes: the mind-body state intervention apparatus according to any one of Techniques 1 to 11; the physiological amount detector; and the intervention equipment.

### [Technique 13]

A mind-body state intervention method is an information processing method to be executed by a computer. The mind-body state intervention method includes: acquiring a physiological amount of a subject detected by a physiological amount detector; quantifying, for a predetermined mind-body state, two mind-body states based on the physiological amount, the two mind-body states including a mind-body state that the subject is aware of and a mind-body state that the subject is unaware of; and performing intervention control in which, with reference to relational data that indicates each of a plurality of intervention control schemes and intervention effects on the two mind-body states quantified, one intervention control scheme is selected from among the plurality of intervention control schemes, based on the intervention effect on at least one of the two mind-body states quantified, and intervention equipment is controlled in accordance with the one intervention control scheme selected.

### [Reference Signs List]

100 mind-body state intervention apparatus according to Embodiment 1 (mind-body state intervention apparatus)
200 mind-body state intervention apparatus according to Variation 1 of Embodiment 1 (mind-body state intervention apparatus )
300 mind-body state intervention apparatus according to Variation 2 of Embodiment 1 (mind-body state intervention apparatus )
400 mind-body state intervention apparatus according to Embodiment 2 (mind-body state intervention apparatus)
500 mind-body state intervention apparatus according to Embodiment 3 (mind-body state intervention apparatus)
600 mind-body state intervention apparatus according to Embodiment 4 (mind-body state intervention apparatus)
700 mind-body state intervention apparatus according to Embodiment 5 (mind-body state intervention apparatus)
110 physiological amount acquirer
11 physiological amount acquirer
120 quantification processor
12 quantification processor
130 first storage (storage)
140 intervention controller
150 intervention equipment
160 physiological amount detector
16 physiological amount detector
17 intervention effect calculator
21 workload acquirer
22 work efficiency calculator
310 mode selector
320 mode acquirer

## Claims

1. A mind-body state intervention apparatus comprising:
a physiological amount acquirer that acquires a physiological amount of a subject detected by a physiological amount detector;
a quantification processor that quantifies, for a predetermined mind-body state, two mind-body states based on the physiological amount, the two mind-body states including a mind-body state that the subject is aware of and a mind-body state that the subject is unaware of;
a storage that stores relational data in advance, the relational data indicating relationships between each of a plurality of intervention control schemes and intervention effects on the two mind-body states quantified; and
an intervention controller that performs intervention control in which, with reference to the relational data, one intervention control scheme is selected from among the plurality of intervention control schemes, based on the intervention effect on at least one of the two mind-body states quantified by the quantification processor, and intervention equipment is controlled in accordance with the one intervention control scheme selected.

2. The mind-body state intervention apparatus according to claim 1, further comprising:
a mode acquirer that acquires a mode from a mode selector, the mode selector prompting the subject to select either a mode that prioritizes an improvement in the intervention effects or a mode that prioritizes an improvement in work efficiency, the work efficiency being efficiency of work performed by the subject,
wherein the relational data further indicates relationships between each of the plurality of intervention control schemes and the work efficiency, and
the intervention controller selects an intervention control scheme based on the intervention effects, the work efficiency, and the mode.

3. The mind-body state intervention apparatus according to claim 1 or 2, further comprising:
a relational data creation device that creates the relational data from a plurality of subjects as targets and stores the relational data in the storage.

4. The mind-body state intervention apparatus according to claim 3,
wherein the relational data creation device includes:
a workload acquirer that acquires a volume of work to be performed by the subject, the volume being detected by a workload detector;
a work efficiency calculator that calculates work efficiency based on the volume of work; and
an intervention effect calculator that calculates the intervention effects, and
the relational data creation device stores the work efficiency calculated and the intervention effects calculated as the relational data in the storage.

5. The mind-body state intervention apparatus according to claim 2,
wherein, when the mode selector selects a mode that prioritizes an improvement in the intervention effects, the intervention controller references the relational data to select an intervention control scheme that maximizes the intervention effects from among the plurality of intervention control schemes.

6. The mind-body state intervention apparatus according to claim 2,
wherein, when the mode selector selects a mode that prioritizes an improvement in the work efficiency, the intervention controller references the relational data to select an intervention control scheme that maximizes the work efficiency from among the plurality of intervention control schemes.

7. The mind-body state intervention apparatus according to claim 1 or 2,
wherein the intervention effects are amounts of change in each of the mind-body states before and after intervention control of the subject.

8. The mind-body state intervention apparatus according to claim 2,
wherein the work efficiency is a rate of change in workload per unit time before and after intervention control of the subject, the unit time being a time required for the intervention control.

9. The mind-body state intervention apparatus according to claim 1 or 2,
wherein the intervention controller presents, to the subject, an amount of change in each of the mind-body states before and after intervention control.

10. The mind-body state intervention apparatus according to claim 1 or 2,
wherein the intervention controller presents, to the subject, the one intervention control scheme used in the intervention control.

11. The mind-body state intervention apparatus according to claim 1 or 2,
wherein the mind-body state is fatigue felt by the subject.

12. A mind-body state intervention system comprising:
the mind-body state intervention apparatus according to claim 1;
the physiological amount detector; and
the intervention equipment.

13. A mind-body state intervention method that is an information processing method to be executed by a computer, the mind-body state intervention method comprising:
acquiring a physiological amount of a subject detected by a physiological amount detector;
quantifying, for a predetermined mind-body state, two mind-body states based on the physiological amount, the two mind-body states including a mind-body state that the subject is aware of and a mind-body state that the subject is unaware of; and
performing intervention control in which, with reference to relational data that indicates each of a plurality of intervention control schemes and intervention effects on the two mind-body states quantified, one intervention control scheme is selected from among the plurality of intervention control schemes, based on the intervention effect on at least one of the two mind-body states quantified, and intervention equipment is controlled in accordance with the one intervention control scheme selected.
